# EUROPEAN PATENT SPECIFICATION

(11) **EP 4 159 050 B1**
(45) Date of publication and mention of the grant of the patent: **13.05.2026**
(21) Application number: 22201856.6
(22) Date of filing: 30.11.2016
(51) Int. Cl.: A23L 27/30, A23L 2/60, C07H 15/24

(54) **STEVIOL GLYCOSIDE COMPOSITIONS FOR ORAL INGESTION OR USE**
STEVIOLGLYCOSIDZUSAMMENSETZUNGEN ZUR ORALEN EINNAHME ODER VERWENDUNG
COMPOSITIONS DE GLYCOSIDES DE STÉVIOL POUR INGESTION OU UTILISATION ORALE

(30) Priority: 30.11.2015 US 201562260942 P; 17.06.2016 US 201662351674 P
(43) Date of publication of application: 05.04.2023
(62) Divisional of application: 16871428.5
(73) Proprietor: Cargill, Incorporated, Wayzata, MN 55391 (US)
(72) Inventor: CARLSON, Ting Liu, Marietta (US); GASPARD, Dan S., Victoria (US); GUTHRIE, Brian D., Chanhassen (US); MORTENSON, Michael Alan, Rogers (US)
(74) Representative: Forresters IP LLP

(56) References cited:
- WO-A1-2014/052457
- WO-A1-2014/086890
- WO-A1-2016/120486
- WO-A2-2014/122227
- US-A1- 2013 309 389
- US-A1- 2014 342 043
- INDRA PRAKASH ET AL: "Development of Next Generation Stevia Sweetener: Rebaudioside M", FOODS, vol. 3, no. 1, 27 February 2014 (2014-02-27), CH, pages 162 - 175, XP055309887, ISSN: 2304-8158, DOI: 10.3390/foods3010162

## Description

### FIELD

The present disclosure relates to compositions including a plurality of steviol glycosides. The present disclosure also relates to sweetener compositions and sweetened compositions including a combination of distinct steviol glycosides, and a method of making a sweetener composition suitable for oral ingestion or oral use, and uses of such sweetener compositions to prepare sweetened compositions including food, beverages, dental products, pharmaceuticals, nutraceuticals, and the like.

The invention is restricted to the subject-matter of the claims.

### BACKGROUND

Sugars, such as sucrose, fructose and glucose, are utilized to provide a pleasant taste to beverages, foods, pharmaceuticals, and oral hygienic/cosmetic products. Sucrose, in particular, imparts a taste preferred by consumers. Although sucrose provides superior sweetness characteristics, it is caloric. Non-caloric or lower caloric sweeteners have been introduced to satisfy consumer demand, and there is desire for these types of sweeteners that have favorable taste characteristics.

Stevia is a genus of about 240 species of herbs and shrubs in the sunflower family (*Asteraceae*), native to subtropical and tropical regions from western North America to South America. The species *Stevia rebaudiana,* commonly known as sweetleaf, sweet leaf, sugarleaf, or simply stevia, is widely grown for its sweet leaves. Stevia-based sweeteners may be obtained by extracting one or more sweet compounds from the leaves. Many of these compounds are steviol glycosides, which are glycosides of steviol, a diterpene compound. These diterpene glycosides are about 150 to 450 times sweeter than sugar.

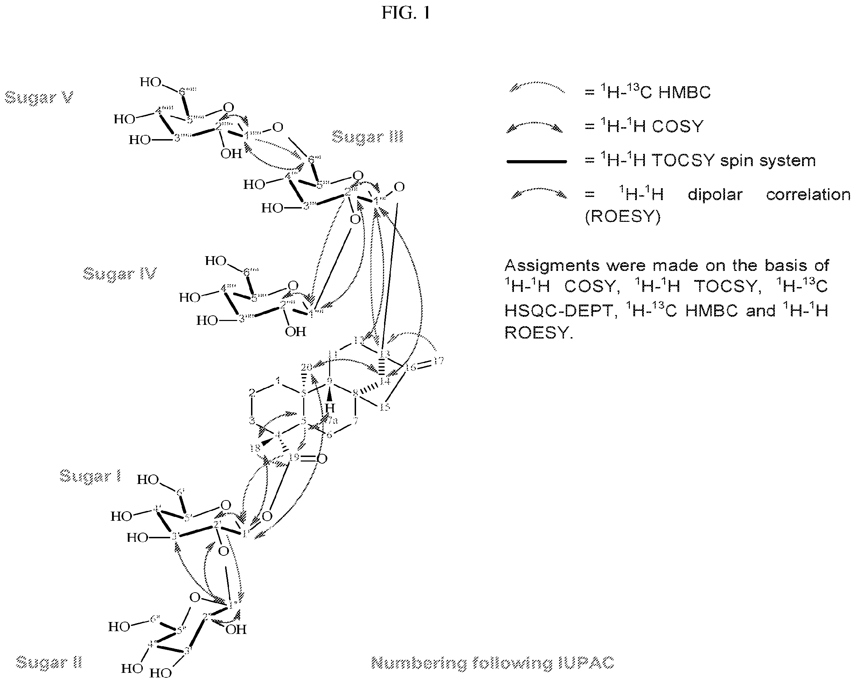

Examples of steviol glycosides are described in published International application WO 2013/096420 (see, e.g., the listing in Fig. 1); and in Ohta et al., "Characterization of Novel Steviol Glycosides from Leaves of Stevia rebaudiana Morita," J. Appl. Glycosi., 57, 199-209 (2010) (See, e.g., Table 4 at p. 204). Structurally, the diterpene glycosides are characterized by a single base structure, steviol, and differ by the presence of carbohydrate residues at positions C13 and C19. See also PCT Patent Publication WO 2013/096420.

Typically, on a dry weight basis, the four major steviol glycosides found in the leaves of *Stevia* are dulcoside A (0.3%), Reb C (0.6-1.0%), Reb A (3.8%) and stevioside (9.1%). Other glycosides identified in *Stevia* extract include one or more of Reb B, D, E, F, G, H, I, J, K, L, M, N, O, steviolbioside and rubusoside.

While the major steviol glycoside Reb A is commonly used as sweetener in beverage applications it has off-taste issues. More recently, there has been focus on certain minor steviol glycosides which have better taste properties. For example, Reb M has higher sweetness intensity and is more potent than other steviol glycosides (e.g., see Prakash, I., et al. (2013) Nat. Prod. Commun., 8: 1523-1526, and WO 2013/096420). Reb D tastes about 200-220 times sweeter than sucrose and in a sensory evaluation it had a slow onset of sweetness and was very clean (e.g., see Prakash, I., et al. (2012) Int. J. Mol. Sci., 13:15126-15136).

Some minor rebaudiosides can be challenging to use because they have less than desirable water solubility properties. For example, it has been reported that Reb D is difficult to use in food products because of its low solubility in water at room temperature. For instance, Reb D needs to be heated to near boiling water temperature for 2 hours in order to achieve complete dissolution at 0.8% concentration. At most only 300 to 450 ppm can be solubilized in water at 23° C (e.g., see US 2013/0251881). As another example, Reb M obtained from *Stevia rebaudiana* has poor aqueous solubility and dissolution qualities in beverage formulations (e.g., see US 2014/0171519).

Certain methods to improve rebaudioside solubility are less than desirable because they are labor intensive, requiring high processing temperatures and the use of excipient compounds. For example, see WO 2013/148177.
WO 2016/120486 A1 relates to recombinant microorganisms and methods for producing steviol glycosides, glycosylated ent-kaurenol, and glycosylated ent-kaurenoic acid.
US 2014/342043 A1 discloses sweetener compositions suitable for use in beverage products and other food products.
US 2013/309389 A1 discloses sweetener compositions comprising particular glycoside blends.
WO 2014/086890 A1 discloses materials and methods for producing particular steviol glycosides, and high-purity compositions of particular steviol glycosides with improved sensory profiles.

### SUMMARY

The present invention is defined in the appended claims. In particular, the invention provides a method of making a sweetener composition suitable for oral ingestion or oral use, comprising: adding a sensory modifying amount of one or more of compounds SG201, SG203, or SG204 according to the following structures: 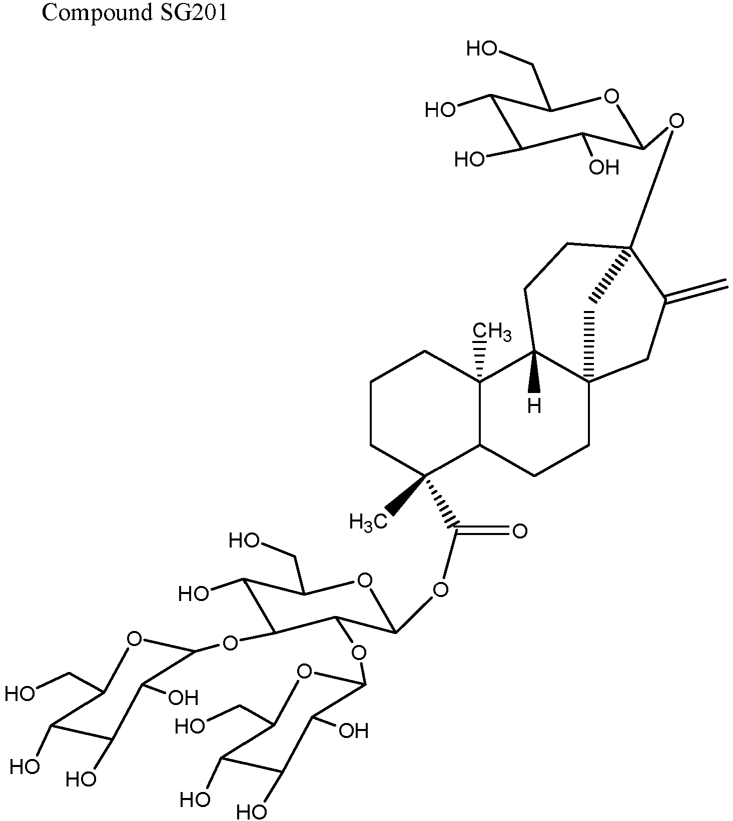 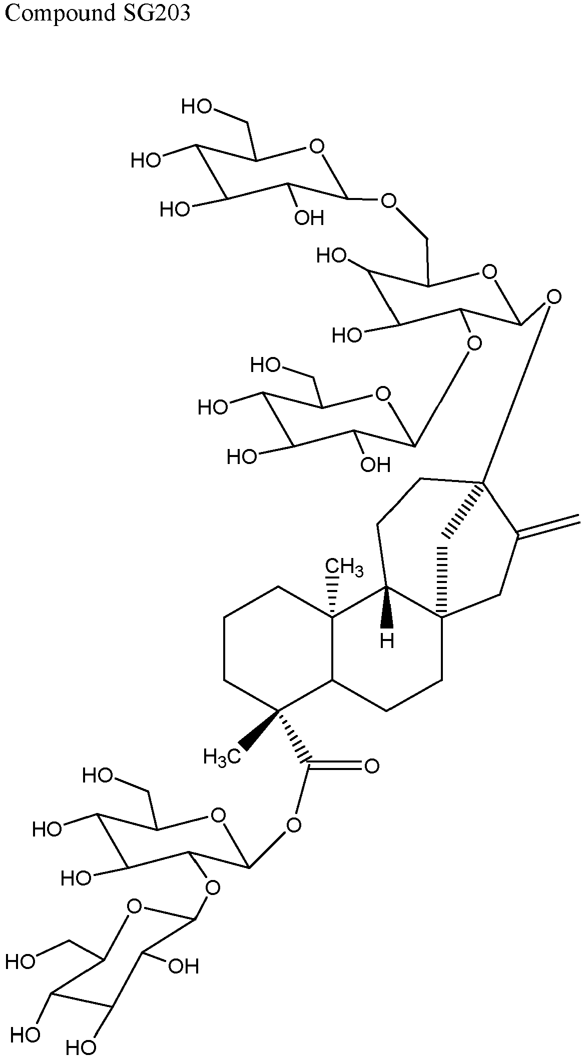 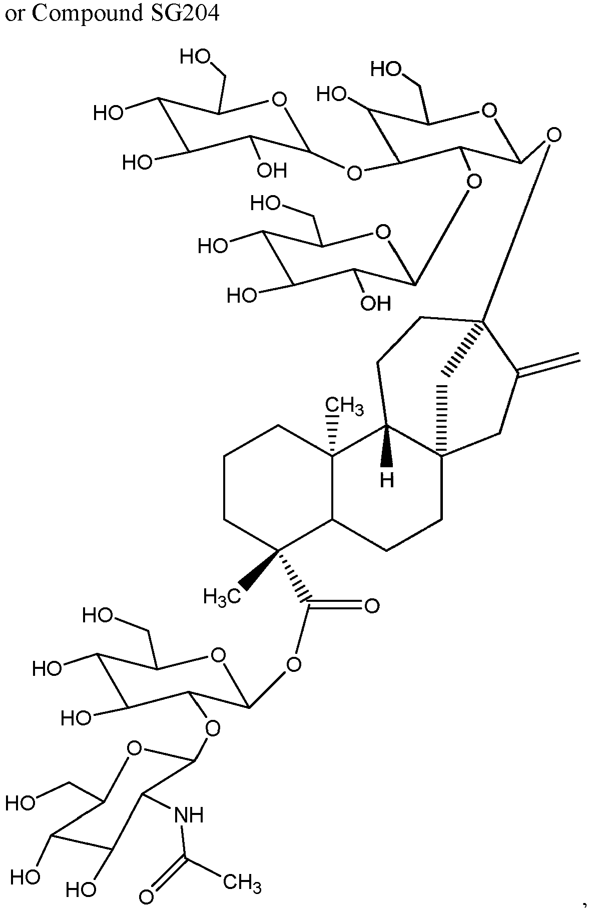 and an amount of one or more of Reb M, Reb D, Reb B or Reb A, wherein a sensory modifying amount of one or more of compounds SG201, SG203, or SG204 is in the range of 0.05 % to 10 % (wt) of a total amount of steviol glycosides in the composition.

In the following, whenever reference is made to the disclosure of or an embodiment including subject-matter not falling in the scope of the claims, this does not pertain to the invention.

The present disclosure generally relates to compositions having a plurality of steviol glycosides. The disclosure also relates to uses of the plurality of steviol glycosides as sweetener compositions, which may be used to prepare sweetened compositions including food, beverages, dental products, pharmaceuticals, nutraceuticals, and the like. In one embodiment, the present disclosure generally relates to sweetener compositions, e.g., a solid composition such as a powder or an aqueous liquid composition (e.g., a concentrate or beverage), having selected combinations of steviol glycosides, including one or more rebaudiosides (Rebs), that are present in specific amounts or concentrations, and uses thereof. The combinations of steviol glycosides may include one or more of dulcoside A, Reb C, Reb A, stevioside, Reb B, Reb D, Reb E, Reb F, Reb G, Reb H, Reb I, Reb J, Reb K, Reb L, Reb M, Reb N, Reb O, steviolbioside, and/or rubusoside (referred to herein as "major steviol glycosides"), and include one or more steviol glycosides that are not one of the major steviol glycosides (referred to herein as SG201, SG202, SG203 and SG204). In one embodiment, each of the SG201, SG203 and SG204, individually or in combination, is present in a (first) product in an amount that provides for sensory modification (a "sensory modifying" amount) relative to a (second) product that lacks one SG201, SG203 and SG204. For example, a product with Reb M, Reb D or Reb M and Reb D, and one or more of compounds SG201, SG203, or SG204 (see below), has at least one different sensory characteristic relative to a product with only Reb M, Reb D or Reb B and Reb D, respectively. In one embodiment, the product is a sweetener composition. The sweetener composition may be used to prepare sweetened compositions including food, beverages, dental products, pharmaceuticals, nutraceuticals, and the like.

A composition or compound that provides for sensory modification may be referred to as a sensory modifier. A sensory modifier changes the sensory characteristics of a sweetened consumable, e.g., a sweetener composition, a beverage, a food product, and the like. Non-limiting examples of sensory characteristics that a sensory modifier can change include bitterness, sourness, numbness, astringency, metallic-ness, cloyingness, dryness, sweetness, temporal aspects of sweetness, as well as flavor notes such as licorice, vanilla, prune, cotton candy, and molasses flavor notes. The sensory modifier may enhance a sensory characteristic, such as enhancing sweetness; may suppress a sensory characteristic, such as reducing bitterness; or may change the temporal aspects of a sensory characteristic, e.g., by reducing sweetness lingering.

In one embodiment, the composition includes a sensory modifying amount of one or more of compounds SG201, SG203, and SG204, having the following structures:

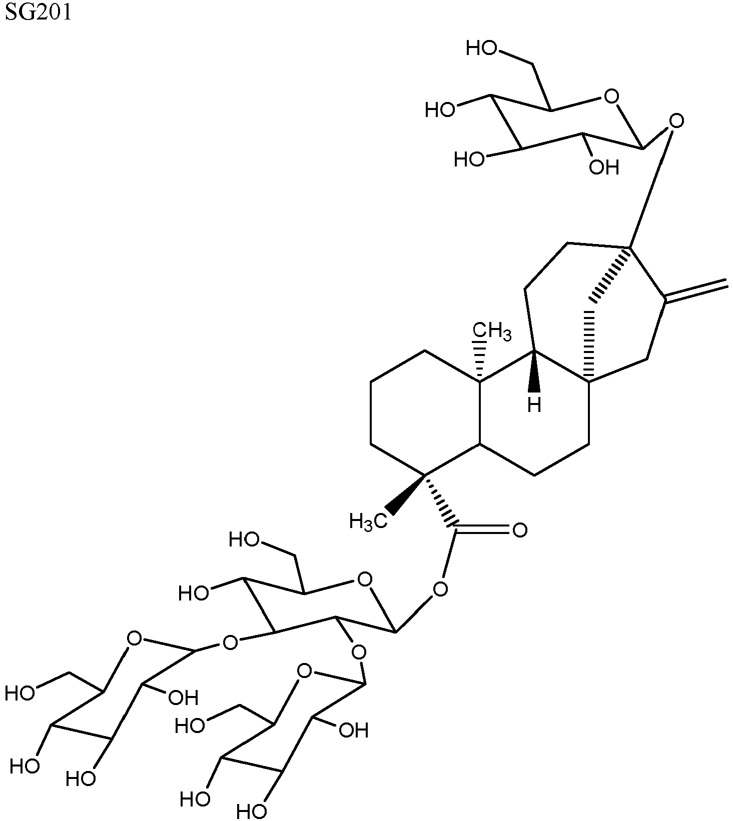

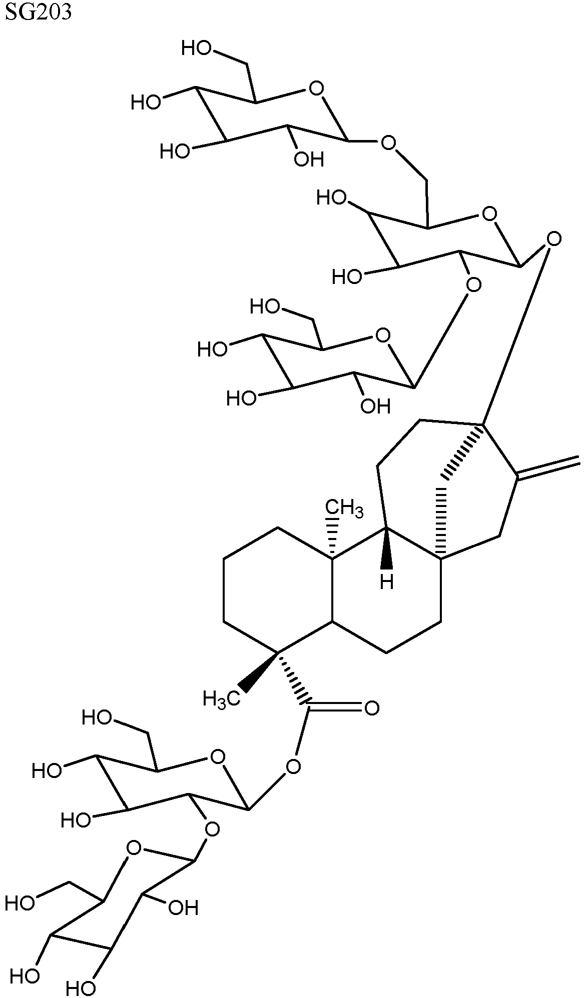

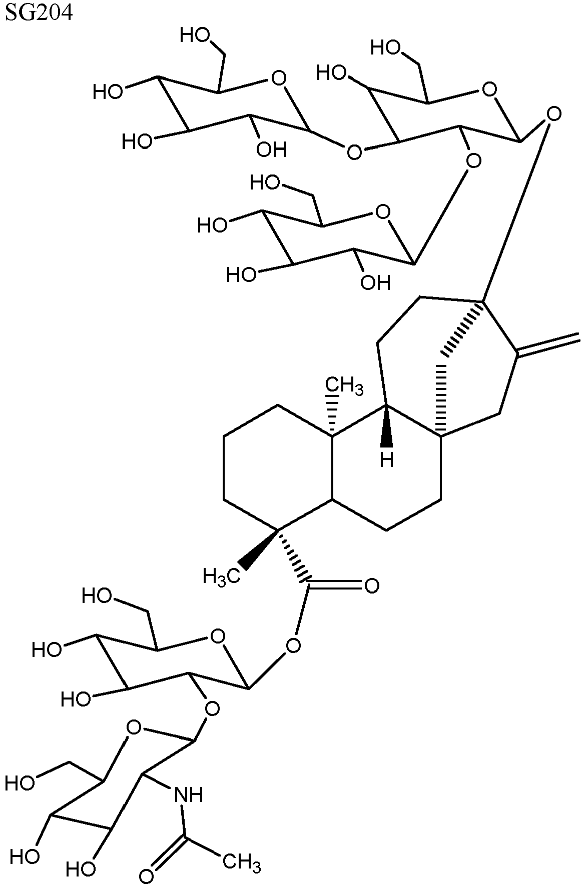

Compounds SG201-204 may be obtained, individually, in isolated and purified form. The isolated compound(s) may then be combined with other compounds, including other steviol glycosides. Compounds SG201-204 may also be produced in a mixture with each other and optionally with other steviol glycosides or other components. Thus, in some embodiments, a mixture of one or more of SG201, SG203 or SG204, may be purified from the other steviol glycosides or other components, or the mixture can include one or more other component(s), such as other steviol glycosides (e.g., Reb M and/or Reb D), that are different from compounds SG201, SG203, and SG204.

Accordingly, other embodiments are directed to sweetener compositions comprising a sensory modifying amount of one or more of compound(s) SG201, SG203, or SG204, with one or more other component(s), such as other steviol glycosides, e.g., rebaudioside M, rebaudioside D, rebaudioside A and/or rebaudioside B, or other sweeteners, e.g., non-nutritive sweeteners or nutritive sweeteners such as erythritol, maltose, honey, sucrose, and the like. In one embodiment, one or more of compounds SG201, SG203, or SG204 and one or more other component(s), such as other steviol glycosides, e.g., major steviol glycosides including rebaudioside M, rebaudioside D, rebaudioside A and/or rebaudioside B, are in a sweetener composition. In one embodiment, one or more of compound(s) SG201, SG203, or SG204 and two or more other component(s), such as other steviol glycosides, e.g., major steviol glycosides including rebaudioside M, rebaudioside D, rebaudioside A and/or rebaudioside B, are in a sweetener composition. In one embodiment, one or more of compounds SG201, SG203, or SG204 and one or more other component(s), such as sweeteners other than steviol glycosides, e.g., non-nutritive sweeteners or nutritive sweeteners such as erythritol, maltose, sucrose, honey and the like, can be used in a beverage. In one embodiment, one or more of compound(s) SG201, SG203, or SG204 can be used in a beverage.

Other embodiments are directed to methods of making a sweetener composition suitable for oral ingestion or oral use. The method includes adding a sensory modifying amount of one or more of compounds SG201, SG203, or SG204, along with one or more other steviol glycosides (e.g., rebaudioside M, rebaudioside D, rebaudioside A and/or rebaudioside B), or other sweeteners, to a material or composition suitable for oral ingestion or use, wherein a sensory modifying amount of one or more compounds of SG201, SG203, or SG204 is in the range of 0.05% to 10% (wt) of a total amount of steviol glycosides in the composition. Accordingly, a composition is disclosed that is suitable for oral ingestion or oral use comprising one or more of compounds SG201, SG203, or SG204 along with one or more other steviol glycosides or other sweeteners, either non-nutritive sweeteners or nutritive sweeteners, a composition such as beverages, beverage concentrates, frozen beverage, powders, foodstuffs, confections, condiments, chewing gum, dairy products, sweeteners, pharmaceutical compositions, and dental compositions. Other embodiments of a method of making a sweetener composition suitable for oral ingestion or oral use include adding a sensory modifying amount or one or more of compounds SG201, SG203, or SG204 along with one or more other steviol glycosides or other sweeteners, either non-nutritive sweeteners or nutritive sweeteners. In one embodiment, a sweetener composition has one or more of SG201, SG203, or SG204 present in an amount corresponding to a sucrose equivalent value (SEV) of less than about 1.5, less than about 1.0 or less than about 0.5. In one embodiment, a sweetener composition has one or more of SG201, SG203, or SG204 present in an amount corresponding to a sucrose equivalent value of greater than about 1.5, greater than about 3, greater than about 5 or more.

In some embodiments, a composition including one or more of SG201, SG203, or SG204 can be used as a sweetener, i.e., one or more of compounds SG201, SG203, or SG204 are used at a concentration resulting in a SEV greater than 1.5 in a beverage or other sweetened composition. In some embodiments, a composition including one or more of SG201, SG203, or SG204 has a SEV of greater than about 5, 6, 7, 8, 9, or 10 when used at a concentration of 1,500 ppm or less, 1,000 or less, 800 or less, 600 or less, 500 or less, 400 or less, 300 or less, or 200 or less.

Yet another embodiment is directed to fermentation media comprising one or more of compound(s) SG201, SG203, SG204, optionally with one or more other component(s), such as other steviol glycosides, e.g., Reb M and/or Reb D. A recombinant host cell can be used to metabolically produce one or more of compound(s) SG201, SG203, SG204. The fermentation media can be enriched in those steviol glycosides or refined to select for certain steviol glycosides.

In one embodiment, an aqueous or solid composition is provided having one or more of rebaudioside A, rebaudioside B, rebaudioside M, rebaudioside D, rebaudioside I, rebaudioside Q, rebaudioside N, or stevioside, and one or more of compounds SG201, SG203, SG204. At least one of the glycosides in the composition has a higher or equal molecular weight than rebaudioside M. In one embodiment the composition is a sweetener composition. In one embodiment, the composition is a beverage. In one embodiment, the pH of the beverage that includes at least one of SG201, SG203 or SG204 may be in the range of 1.8 to 10, 2 to 5, or 2.5 to 4.2.

In one embodiment, an aqueous or solid composition is disclosed having one or more of rebaudioside A, rebaudioside B, rebaudioside M, rebaudioside D, and one or more of compounds SG201, SG203, or SG204. In one embodiment, an aqueous or solid composition is disclosed having Reb M, Reb D or Reb M and Reb D, and one or more of compounds SG201, SG203, or SG204. In one embodiment the composition is a sweetener composition. In one embodiment, the composition is a beverage. In one embodiment, the pH of the beverage that includes one or more of compounds SG201, SG203, or SG204 may be in the range of 1.8 to 10, 2 to 5, or 2.5 to 4.2.

In one embodiment, the composition is a beverage and the total glycoside content in the beverage is about 50 to 1500 ppm, 100 to 1200 ppm, 200 to 1000 ppm, 300 to 900 ppm, 350 to 800 ppm, 400 to 600 ppm, 350 to 550 ppm, or 450 to 550 ppm. In one embodiment, one or more of compounds SG201-204 are present in a beverage in a range of about 0.011 ppm to about 1000 ppm, e.g., about 50 ppm to about 500 ppm, 10 to 400 ppm, 50 to 200 ppm, 75 to 150 ppm, 5 to 200 ppm, 10 to 100 ppm, 1 to 100 ppm, 20 to 90 ppm, 30 to 80 ppm, 40 to 70 ppm, 45 to 55 ppm, 0.1 to 50 ppm, 0.1 to 40 ppm, 0.1 to 30 ppm, 0.1 to 20 ppm, 0.1 to 10 ppm, 1 to 10 ppm, 1 to 5 ppm, 0.01 to 100 ppm, 0.01 to 10 ppm, or 0.1 to 1 ppm. In some embodiments, one or more of SG201, SG203, or SG204 are present in a beverage or other sweetened composition in an amount including at least 0.001, 0.01, 0.1, 1, 5, 10, 20, 30, 40, 50, 60, 70, 80, 90, 100, 125, 150, 175, or 200 ppm.

In one embodiment, steviol glycosides other than Reb D, Reb M, Reb G, Reb O, Reb N, and/or Reb E, or other than Reb D, Reb M, Reb B and/or Reb A, or other than Reb B and/or Reb D, including for example one or more of compounds SG201, SG203, or SG204 are present in a sweetener composition at about 0.01 to 100 wt% of the total glycoside content of the sweetener composition. In other embodiments, one or more of compounds SG201, SG203, or SG204 are present in a sweetener composition at about 0.05 to 70 wt%, e.g., about 0.1 to 50 wt%, 0.5 to 70 wt%, 1 to 50 wt%, 1 to 35 wt%, 2 to 25 wt%, 3 to 20 wt%, 5 to 15 wt%, 0.1 to 15 wt%, 0.5 to 10 wt%, or 1 to 5 wt%. In other embodiments, one or more of compounds SG201, SG203, or SG204 are present in a sweetener composition or beverage at about 0.01 to 100 wt%, e.g., about 0.05 to 70 wt%, 0.1 to 50 wt%, 0.5 to 70 wt%, 1 to 50 wt%, 1 to 35 wt%, 2 to 25 wt%, 3 to 20 wt%, 5 to 15 wt%, 0.1 to 15 wt%, 0.5 to 10 wt%, or 1 to 5 wt%, of the total steviol glycoside content of the composition. In one embodiment, steviol glycosides other than Reb D, Reb M, Reb G, Reb O, Reb N, and/or Reb E, or other than Reb D, Reb M, Reb B and/or Reb A, or other than Reb B and/or Reb D, for example one or more compounds SG201, SG203, or SG204, are at a weight ratio of the total of all other glycosides in a liquid composition, such as a beverage or concentrate, or a dry solid, of 1:1 to 1:20, 1:1.5 to 1:15, 1:2 to 1:10, 1:2.5 to 1:7.5, or 1:3 to 1:5.

Steviol glycosides can also be included in a concentrated syrup that can be used to make a beverage, also referred to as a "throw syrup." In some embodiments, the steviol glycoside content is 2 to 10, 3 to 7, 4 to 6, or about 5 times greater in the syrup concentrate than the desired concentration of the finished beverage. Accordingly, the total steviol glycoside content, the content of any single major steviol glycoside, and/or the content of any one of SG201, SG203, SG204, in a syrup concentrate can be in the range of about 100 to 15,000 ppm, 500 to 12,500 ppm, 1,000 to 10,000 ppm, 1,500 to 7,500 ppm, 2,000 to 6,000 ppm, 2,000 to 4,200 ppm, or 2,400 to 3,600 ppm. In some embodiments, the content of any of compounds SG201, SG203, or SG204, or the total content of the combination of compounds SG201, SG203, SG204, in the syrup concentrate is at least 5 ppm, 25 ppm, 50 ppm, 100 ppm, 150 ppm, 200 ppm, 250 ppm, 500 ppm, 750 ppm, or 1,000 ppm.

Other embodiments of the disclosure are directed to providing or enhancing sweetness to a composition suitable for oral ingestion or oral use comprising adding one or more of the compounds SG201, SG203, or SG204, such as along with one or more other steviol glycosides (e.g., Reb M and/or Reb D), to a material or composition suitable for oral ingestion or use. Accordingly the disclosure also provides a composition suitable for oral ingestion or oral use comprising one or more of the compounds SG201, SG203, or SG204 along with two or more other steviol glycosides or other sweeteners, either non-nutritive sweeteners or nutritive sweeteners, compositions such as beverages, beverage concentrates, frozen beverage, powders, foodstuffs, confections, condiments, chewing gum, dairy products, sweeteners, pharmaceutical compositions, and dental compositions. Other embodiments include a composition suitable for oral ingestion or oral use comprising two or more of the compounds SG201, SG203, or SG204 along with one or more other steviol glycosides or other sweeteners, either non-nutritive sweeteners or nutritive sweeteners.

In another embodiment, there is disclosed a method for enhancing the solubility of a steviol glycoside in an aqueous composition. The method comprises a step of providing an aqueous composition comprising at least first and second steviol glycosides. The second steviol glycoside is different than the first steviol glycoside and has a solubility in an aqueous composition (that lacks the first steviol glycoside) that is lower than its solubility in an aqueous composition that includes the first steviol glycoside. Compounds SG201, SG203, or SG204 may exemplify the first steviol glycoside. As an example, the solubility of the first and second steviol glycosides can be enhanced by producing the first and second glycosides together, such as by a recombinant organism under fermentation conditions. As another example, the solubility of the first and/or second steviol glycosides can be enhanced by adding the first steviol glycoside to a composition that has the second steviol glycoside.

In another embodiment, there is disclosed another method for enhancing the solubility of a steviol glycoside in an aqueous composition. The method includes a step of providing an aqueous composition comprising first and second steviol glycosides, wherein the second steviol glycoside is selected from the group consisting of Reb A, Reb B, Reb M, Reb D, Reb I, Reb Q, Reb N, and stevioside. The first steviol glycoside is different from the second steviol glycoside (such as, for example, having a higher or equal molecular weight than the Reb M), and the second steviol glycoside has a solubility in an aqueous composition that lacks the first steviol glycoside that is lower than a solubility of the second steviol glycoside in an aqueous composition that includes the first steviol glycoside.

In another embodiment, there is disclosed a method for enhancing the solubility of a steviol glycoside in a composition. The method includes a step of providing a composition comprising a first composition having a first steviol glycoside and a second composition having a second steviol glycoside, wherein the second steviol glycoside may be one or more of Reb A, Reb B, Reb M, Reb D, Reb I, Reb Q, Reb N, or stevioside. The first steviol glycoside is different from the second steviol glycoside (such as, for example, having a higher or equal molecular weight than the Reb M), and the second steviol glycoside has a solubility in an aqueous composition that lacks the first steviol glycoside that is lower than a solubility of the second steviol glycoside in an aqueous composition that includes the first steviol glycoside.

In some embodiments, there is disclosed a sweetener composition comprising one or more, two or more, three or more, or all three of compounds SG201, SG203, or SG204. In some such embodiments, the compounds SG201, SG203 and SG204 are present in the composition in a total amount in the range of 0.001 % to 10 % (wt) of a total amount of steviol glycosides in the composition. In some such embodiments, each one of the compounds SG201, SG203 and SG204 are present in the composition in an amount in the range of 0.001 % to 10 % (wt) of a total amount of steviol glycosides in the composition. In some embodiments, there is disclosed a foodstuff or beverage comprising the composition of any of the embodiments of this paragraph.

### DESCRIPTION OF THE FIGURES

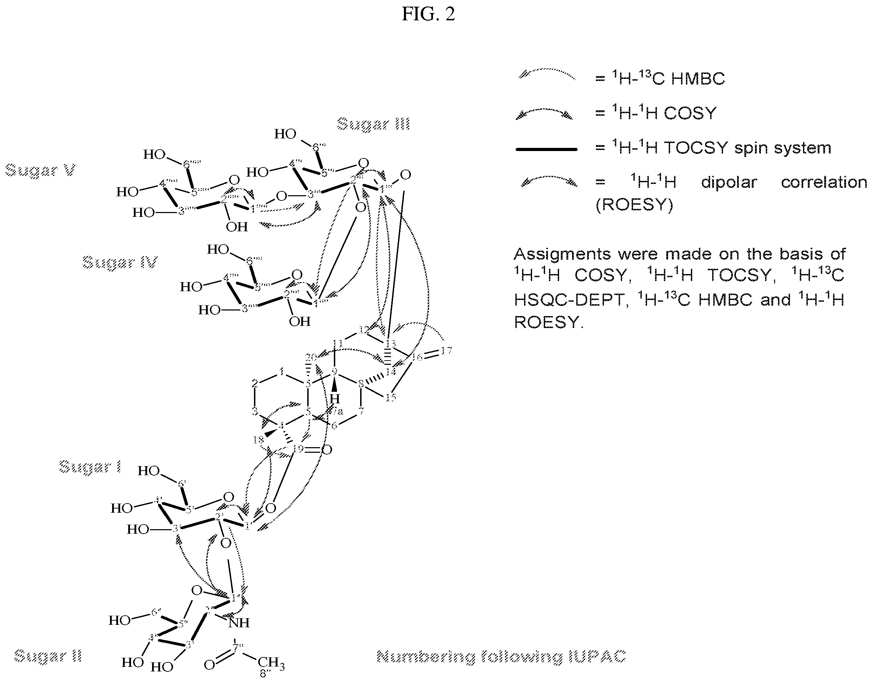

Figures 1 and 2 illustrate spectrophotometric coupling of selected atoms that were used to establish the structures of compounds SG203 and SG204.

### DETAILED DESCRIPTION

Embodiments of the disclosure described herein are not intended to be exhaustive or to limit the invention to the precise forms disclosed in the following detailed description. Rather a purpose of the embodiments chosen and described is so that the appreciation and understanding by others skilled in the art of the principles and practices of the present invention can be facilitated. The invention pertains to a method of making a sweetener composition suitable for oral ingestion or oral use comprising adding a sensory modifying amount of one or more of compounds SG201, SG203, or SG204 as defined in the claims and an amount of one or more of Reb M, Reb D, Reb B, or Reb A, wherein a sensory modifying amount of one or more of compounds SG201, SG203, or SG204 is in the range of 0.05% to 10% of a total amount of steviol glycosides in the composition.

For example, some embodiments of the disclosure are directed to compositions having a sensory modifying amount of one or more of compounds SG201, SG203, or SG204 (having the structures set out above). Thus, in some embodiments, one or more of SG201-204 can be used as a sensory modifier. In one embodiment, one or more of SG201-204, when present in a sweetener composition, beverage, food product, etc., provide for sensory modification are present at a level below a sweetening threshold. In one respect, such a sensory modification may be present in the consumable at a concentration that produces a sucrose equivalent value (SEV) of about 1.5 or less, 1.0 or less, or 0.5 or less in water. For example, one or more of SG201-204 having a SEV of 1.5 or less at a concentration of 500 ppm in water can be a sensory modifier when used at a concentration of 500 ppm or less in a sweetener composition.

In one embodiment, the steviol glycoside is present in an amount that modifies the temporal aspects of a sensory characteristic. The temporal aspects of a sensory characteristic refers to the perception of the characteristic over time. This includes the onset time of the characteristic, i.e., the time it takes to reach peak of the characteristic. It also includes the linger time of the characteristic, i.e., the time from a peak of the sensory characteristic to a level where it is no longer perceived. The temporal aspects may also include a time-intensity profile showing the perceived sweetness as a function of time. These characteristics can all contribute to a temporal profile for the sensory characteristic.

The sweetness temporal profile of sucrose is deemed highly desirable. The sweetness of some non-nutritive sweeteners, including rebaudioside A, is deemed "sharper" than sucrose in that it has a faster sweetness onset, i.e., it reaches the peak sweetness more swiftly and has a shorter onset time. Such fast-onset sweeteners may also be referred to as "spiky" (e.g., artificial). Some non-nutritive sweeteners may have a sweetness that lingers longer than sucrose, i.e., the flavor takes longer to dissipate from peak sweetness to a level where sweetness is no longer perceived. A sweetener composition that has a sweetness temporal profile closer to that of sucrose is deemed more desirable. Thus, in one embodiment, one or more of SG201-204 in a composition provides for enhanced sweetness.

A sensory modifier may also have a synergistic effect on the intensity of a sensory characteristic when used in combination with one or more other compounds. A synergistic effect means that the combination of compounds has an enhanced (more than additive) effect on the sensory characteristic when compared to the sensory characteristic of the compounds separately. As a simple example, if rebaudioside A has a sucrose equivalent value (SEV) of 5 at a concentration of 400 ppm in a beverage and the sensory modifier has an SEV of 1 at a concentration of 400 ppm in the beverage, a 50/50 composition of Reb A and the sensory modifier at 400 ppm in a beverage (i.e., 200 ppm Reb A and 200 ppm of the sensory modifier) would be expected to have a SEV of 3. However, the sensory modifier is deemed to have a synergistic sweetening effect if the beverage has a SEV greater than 3 with Reb A at 200 ppm and the sensory modifier at 200 ppm.

Compounds SG201-204 can be produced by microorganisms engineered for the synthesis of other steviol glycosides.

Structurally, compounds SG201-204 have a central molecular moiety, which is a single steviol base structure, and glucopyranosyl or glucoseamine residues attached to the C13 and C19 atoms of the steviol base, according to the atom numbering on the base shown in Formula 1 below. That is, glucopyranosyl or glucoseamines residues represent groups R₂ and R₁ in the following formula:

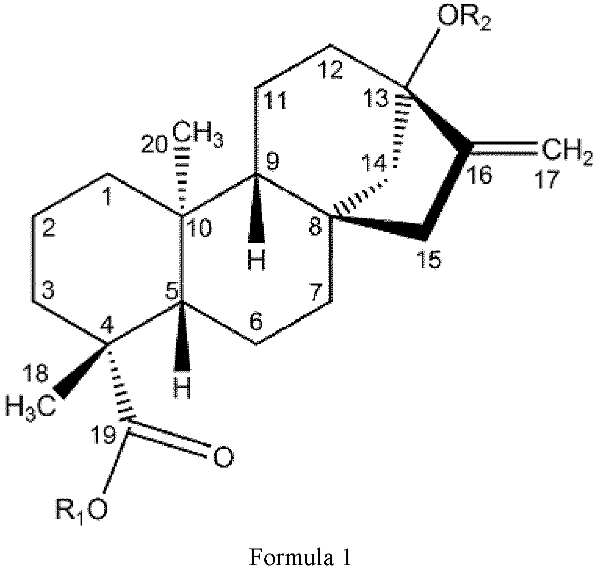

Compound SG201 may be characterized by having a formula weight of 967.01(4) and an exact mass of 966.43078856(4). Compound SG201 has a one glucopyranose residue attached via the number 13 carbon (C13) and a group of 3 glucopyranose residues attached via the number 19 carbon (C19) of the steviol moiety. The group of three glucopyranose residues has a branched (non-linear) structure, meaning that two glucopyranose residues are connected to a single glucopyranose residue.

Compound SG202 may be characterized by having a formula weight of 1453.43(5) and an exact mass of 1452.58925885(7). Compound SG202 has a group of 3 glucopyranose residues attached via the number 13 carbon (C13) and a group of 4 glucopyranose residues attached via the number 19 carbon (C19) of the steviol moiety. Both groups of glucopyranose residues have a branched (non-linear) structure.

Compound SG203 may be characterized by having a group of 3 glucopyranose residues attached via the number 13 carbon (C13) and a group of 2 glucopyranose residues attached via the number 19 carbon (C19) of the steviol moiety. The group of 3 glucopyranose residues at C13 has a branched (non-linear) structure. The spectrophometric couplings of the atoms in compound 3 are illustrated in Figure 1. The assignments of the various couplings were made on the basis of ¹H,¹H-COSY, ¹H,¹H-TCOSY, ¹H,¹H-ROESY, ¹H,¹³C-HSQC-DEPT, and¹H,¹³C-HMBC.

Compound SG204 may be characterized by having a group of 3 glucopyranose residues attached via the number 13 carbon (C13) and a group of two residues of glucopyranose and an acetylated glucose amine derivative attached via the number 19 carbon (C19) of the steviol moiety. The group of 3 glucopyranose residues at C13 has a branched (non-linear) structure. The spectrophometric couplings of the atoms in compound SG204 are illustrated in Figure 1. The assignments of the various couplings were made on the basis of ¹H,¹H-COSY, ¹H,¹H-TCOSY, ¹H,¹H-ROESY, ¹H,¹³C-HSQC-DEPT, and¹H,¹³C-HMBC.

Glucopyranose units in compounds SG201-203 and the glucopyranose and acetylated glucoseamine derivative of compound SG204 of the attached C19 and C13 groups may also be described by their chemical linkages to each other. Chemical linkages of these units include 1→2 glycosidic, 1→3 glycosidic linkage, and 1→6 glycosidic linkages.

In some modes of practice, one or more compounds SG201-204 can be produced in a fermentation process. For example, the fermentation process can use a genetically modified organism that is engineered for the production of one or more steviol glycosides, such as Reb M and Reb D. In particular, one or more compounds SG201-204 can be carried out using an engineered microbial strain having a set of enzymes that provide a pathway for the synthesis of one or more of compounds SG201-204. One or more other steviol glycosides that are different than compounds SG201-204 can also be produced by the engineered microbial strains or enzymatic preparations from the engineered microbial strains.

In one embodiment, an engineered yeast useful for the production of steviol glycosides expresses the following enzymes: geranylgeranyl diphosphate synthase (GGPPS), ent-copalyl diphosphate synthase (CDPS), kaurene oxidase (KO), kaurene synthase (KS); steviol synthase (KAH), cytochrome P450 reductase (CPR), UGT74G1, UGT76G1, UGT91D2, UGT85C2 and a EUGT11. WO 2014/122227 describes an engineered yeast strain that express these enzymes. The UDP-glucosyltransferases can be a gene encoding a polypeptide for example, UGT74G1, UGT85C2, UGT76G1, UGT91D2, and a EUGT11; these genes encode polypeptides capable of carrying out a number of reactions such as a) a gene encoding a polypeptide capable of beta 1,2 glucosylation of the C2' of the 19-O glucose of a steviol glycoside; (b) a gene encoding a polypeptide capable of beta 1,2 glucosylation of the C2' of the 13-*O*-glucose of a steviol glycoside; (c) a gene encoding a polypeptide capable of beta 1,3 glucosylation of the C3' of the 19-*O*-glucose of a steviol glycoside; (d) a gene encoding a polypeptide capable of beta 1,3 glucosylation of the C3' of the 13-*O*-glucose of a steviol glycoside; (i) a gene encoding a polypeptide capable of glucosylation of the 13-OH of steviol or a steviol glycoside;(j) a gene encoding a polypeptide capable of glucosylation of the C-19 carboxyl of steviol or a steviol glycoside. For example, UGT85C2 carries out reaction (i); UGT74G1 carries out reaction (j); UGT91D2 carries out reactions (a; weakly), (b); UGT76G1 carries out reactions (c) and (d); EUGT11 carries out reactions (a), (b; less well).

In one embodiment, an engineered yeast useful for the production of steviol glycosides includes the following genes: a gene encoding a polypeptide capable of beta 1,2 glycosylation of the C2' of the 13-*O*-glucose, 19-*O*-glucose, or both 13-*O*-glucose and 19-*O-*glucose of a steviol glycoside, e.g., UGT91D2 and EUGT11; a gene encoding a polypeptide capable of glycosylation of the 13-OH of steviol or a steviol glycoside, e.g., UGT85C2; a gene encoding a polypeptide capable of beta 1,3 glycosylation of the C3' of the 13-*O*-glucose, 19-*O-*glucose, or both 13-*O*-glucose and 19-*O*-glucose of a steviol glycoside, e.g., UGT76G1; a gene encoding a polypeptide capable of glycosylation of the C-19 carboxyl of steviol or a steviol glycoside, e.g., UGT74G1; a gene encoding a polypeptide capable of synthesizing geranylgeranyl pyrophosphate (GGPP) from farnesyl diphosphate (FPP) and isopentenyl diphosphate (IPP), e.g., geranylgeranyl diphosphate synthase (GGPPS); a gene encoding a polypeptide capable of synthesizing ent-copalyl diphosphate from GGPP, e.g., ent-copalyl diphosphate synthase (CDPS); a gene encoding a polypeptide capable of synthesizing ent-kaurene from ent-copalyl pyrophosphate, e.g., kaurene synthase (KS); gene encoding a polypeptide capable of synthesizing ent-kaurenoic acid from ent-kaurene, e.g., kaurene oxidase (KO); gene encoding a polypeptide capable of synthesizing steviol from ent-kaurenoic acid, e.g., steviol synthase (KAH); and a gene encoding a polypeptide capable of converting NADPH to NADP+, e.g., cytochrome P450 reductase (CPR).

Fermentation can be carried out under conditions and in medium suitable for production of one or more of compounds SG201-204. Other steviol glycosides can be produced by the engineered microbe, such as Reb M, Reb D, Reb A, and Reb B. One or more of compounds SG201-204 can be produced in amounts less than the amounts of steviol glycosides such as Reb M and Reb D. Fermentation conditions generally use oxygen (aerobic conditions), a lower pH, a carbon source, and a nutrient (nitrogen) base. Fermentation can be carried out using a fed batch or continuous process.

Fermentation can be carried out using a first growth phase in base medium, followed by a longer feeding phase using a glucose-containing defined feed medium (with trace metals, vitamins, and salts). The fermentation minimal medium includes glucose (5g/L), ammonium sulfate (5g/L), potassium dihydrogenphosphate (3g/L), magnesium sulphate (0.5 g/L), trace elements, and vitamins (e.g., see, Verduyn, C. et al. (1992) Yeast 8, 501-517). The pH of the fermentation media can be kept at about pH 5 and the temperature at about 30°C.

Optionally, fermentation can be carried out in media containing steviol(s). Using this media, the microorganism contains and expresses genes encoding a functional EUGT1 1, a functional UGT74G1, a functional UGT85C2, a functional UGT76G1, and a functional UGT91 D2. Compounds SG201-204, Reb A, Reb D, and Reb M may be obtained from the fermentation media.

In other modes of practice, one or more of compounds SG201-204 may be isolated in a fermentation broth. For example, methanol may be added to a fermentation broth to make a solution that has 55% methanol (v/v). The resulting solution is then mixed for approximately 30 minutes. The solution is then filtered using a Buchner funnel through a 0.45 µm nylon filter paper to clarify. The filtrate is dried under nitrogen at room temperature to complete dryness. The resulting precipitate was solubilized in 20% ethanol (v/v) and purified on an Agilent 1260 semi-preparative HPLC instrument utilizing a Phenomenex Kinetex ^{®} XB-C18 µm column. An ultrapure water (18.2 MΩ) and methanol gradient was used to separate the desired isomer from the matrix and desired purity was achieved through an iterative approach of compound purification. Similar fractions were pooled and dried under nitrogen at room temperature prior to any further processing.

As another option, preparation of one or more of compounds SG201-204 can be carried out using an enzyme preparation from one or more genetically engineered organism(s), such as an organism described herein. For example, in one mode of practice, a genetically engineered microbe expressing geranylgeranyl diphosphate synthase (GGPPS), ent-copalyl diphosphate synthase (CDPS), kaurene oxidase (KO), kaurene synthase (KS); steviol synthase (KAH), cytochrome P450 reductase (CPR), UGT74G1, UGT76G1, UGT91 d2, UGT85C2, and EUGT11 enzymes is used to make an enzyme composition. For example, the organism can be treated with reagents that disrupt cell membranes to release the enzymes into a composition, or if enzymes are secreted into a growth media for the organism, the media can be used to prepare the composition. The enzyme-containing composition is then contacted with one or more precursor compounds (e.g., a steviol glycoside precursor) which is subjected to at least one enzymatic reaction, or typically multiple enzymatic reactions through a series of intermediates, to provide a composition that includes one or more of compounds SG201-204.

Alternatively, an enzyme composition is prepared by combining cellular extracts from multiple engineered organisms, each organism expressing less than a desired number of enzymes (e.g., one or two) for the enzymatic conversion of a steviol glycoside precursor to one or more of compounds SG201-204. Extracts from the multiple organisms can be combined for preparation of the enzymatic composition.

Following a period of fermentation a composition containing steviol glycosides including one or more of compounds SG201-204 can be obtained from the culture media using various techniques. In some embodiments, a compound such as permeabilizing agent can be added to the fermentation media to enhance removal of the steviol glycosides from the cell and into the media.

The fermentation media can then be centrifuged or filtered to remove the engineered cells. The fermentation media can optionally be treated to remove low molecular weight components (glucose, basic nutrients, and salts), such as by membrane dialysis. Depending on a desired use, a composition comprising one or more of compounds SG201-204, optionally with other steviol glycosides, can be used.

If it is desired to provide a composition with steviol glycosides including compounds SG201-204 in enriched or purified form, or where one or more of compounds SG201-204 are separated from other steviol glycosides, or separated from one another, further purification can be carried out. Such enrichment or purification of steviol glycoside components can be carried out on liquid fermentation media, or the fermentation media can then be dried down prior to purification. For example, fermentation media can be dried down using lyophilization to form a dry composition (e.g., powder or flakes) including steviol glycosides with one or more of compounds SG201-204 that can be subsequently processed.

In some modes of practice, dried fermentation broth enriched for steviol glycosides including one or more of compounds SG201-204, is used as the starting material for purification. For example, a solvent or solvent combination can be added to the dried fermentation broth to dissolve or suspend material that includes the steviol glycosides. An exemplary combination for dissolving the steviol glycosides is a mixture of water and an alcohol (e.g., 50:50 ethanol: water). To facilitate dissolving or suspending, the dried broth materials can be heated at a temperature above room temperature, such as in the range of 40°C - 60°C. Mechanical disruption of the dried broth materials can also be performed, such as by sonication. The dissolved or suspended broth materials can be filtered using a micron or sub-micron prior to further purification, such as by preparative chromatography.

Dried fermentation broth enriched for steviol glycoside compounds can be subjected to purification, such as by reverse phase liquid chromatography. A suitable resin can be used to retain steviol glycoside compounds in the column, with removal of hydrophilic compounds which get washed through the column with a liquid such as water. Elution of steviol glycosides including one or more of compounds SG201-204 from the column can be accomplished a suitable solvent or solvent combination such as acetonitrile or methanol.

Elution of steviol glycosides including one or more of compounds SG201-204 from a reverse phase column can yield a composition which can be useful for any one of a variety of purposes. For example, a purified composition with one or more of compounds SG201-204 can be used as a sweetener composition for oral ingestion or oral use. The composition can be defined with regards to the steviol glycosides in the composition.

For example, one or more of compounds SG201-204 may be defined with regards to the "total steviol glycosides" present in the composition. The "total steviol glycosides" refers all the steviol glycosides present in the composition, including steviol glycosides compounds SG201-204, and steviol glycosides that are different than compounds SG201-204. Total steviol glycosides can be defined in terms of steviol glycoside type and amount.

Exemplary steviol glycosides that are different than compounds SG201-204 include, but are not limited to, Reb M, Reb D, Reb A, Reb B, Reb N, and stevioside. These other steviol glycosides may be produced in a fermentation process along with one or more of compounds SG201-204. The amounts of steviol glycosides in the composition can be expressed in relation to one another, or to the total amount of steviol glycosides, such as by a weight percentage of the total amount of steviol glycosides, or a ratio, or range of ratios, expressed as weight percent, or molar percent.

Total steviol glycosides (TSG) is calculated as the sum of the content of all steviol glycosides in a composition on a dry (anhydrous) basis. Unless expressed herein otherwise, an "amount" of steviol glycoside will refer to the percentage by weight (% wt) of the steviol glycoside, or combination thereof.

In some preparations, any one of compounds SG201-204 is present in the composition in the range of about 0.05 % to about 5 % (wt) of the total amount steviol glycosides in the composition.

The combined amount of compounds SG201-204 can also be expressed in relation to the total amount steviol glycosides in the composition. For example the combined amount of compounds SG201-204, may be present in the range of about 0.01 to 50%, about 0.05 to 40%, about 0.1 to 25%, about 0.5% to about 10%, about 1% to about 8%, about 2% to about 7%, about 4% to about 6%, about 0.001 to 10%, about 0.001 to 5%, about 0.001 to 1%, or about 0.1 to 3%, of the total amount steviol glycosides in the composition. In one embodiment, combined amounts of compounds SG201-204 may be in the range of 0.001% to 50%, 0.01% to 30%, 0.1 to 10%, 0.5 to 5%, 0.01 to 1%, 0.1 to 5% or 0.15 to 0.25% of the total amount steviol glycosides in the composition or of the total of Reb M and Reb D, or Reb M, Reb D, RebA and Reb B. Any combinations of two or more of SG201-204 can be used in a sweetener composition or sweetened composition, including, e.g., SG201 and SG202; SG201 and SG203: SG201 and SG204; SG202 and SG203; SG202 and SG204; SG203 and SG204; SG201, SG202 and SG203; SG201, SG202 and SG204; SG202, SG203 and SG204; or SG201, SG202, SG203 and SG204. In some embodiments, individual amounts of one or more of SG201-204 may be in the range of 0.001% to 50%, 0.01 to 30%, 0.1 to 10%, 0.5 to 5%, 0.001 to 1%, 0.01 to 5%, 0.1 to 3%, 0.1 to 0.5%, or 0.15 to 0.25% of a sweetener composition or the total glycoside content of the sweetener composition. In some embodiments, SG201-204 can be included in an amount of at least 0.0001%, 0.01%, 0.1%, 0.5%, 1%, 2%, 3%, 5%, 10%, 20%, 30%, 40%, 50%, 60%, 70%, 80%, 90%, or 95%, in a sweetener composition or of the total glycoside content of a sweetener composition.

As discussed herein, the composition can include one or more other steviol glycosides that are different than compounds SG201-204. These other steviol glycosides can be retained in a composition if they are not purified away from the compounds SG201-204. For example, other steviol glycosides can be present along with one or more of compounds SG201-204 if the other steviol glycosides are produced in a common fermentation process. Exemplary steviol glycosides include those such as Reb M, Reb D, Reb A, Reb B, Reb N, and stevioside. In some embodiments, the steviol glycosides Reb M and Reb D can be produced by an engineered organism as the predominant steviol glycosides, and therefore can represent the major portion of the steviol glycosides in the composition that includes one or more of compounds SG201-204. Reb M or Reb D can, in some embodiments, be present in the composition an amount greater than any one of compounds SG201-204. For example, Reb M or Reb D can be present in an amount in the range of about 10 times to about 500 times, about 25 times to about 250 times, or about 50 times to about 200 times greater than any one of compounds SG201-204.

A steviol glycoside composition that includes one or more of compounds SG201-204 can optionally be expressed in terms of amounts of Reb M and Reb D. For example, Reb M and Reb D can be present in the composition in a total amount of about 90 % (wt) or greater, about 92.5 % (wt) or greater, or 95 % (wt) or greater, of a total amount steviol glycosides in the composition. Reb M can be the predominant steviol glycoside in the composition, and can be present, for example, in an amount in the range of about 45 % to about 70 %, about 50 % to about 65 %, or about 52.5 % to about 62.5 % of the total amount steviol glycosides in the composition. Reb D can be in an amount less than Reb M, such as in an amount in the range of about 25 % to about 50 %, about 30 % to about 45 %, or about 32.5 % to about 42.5 % of the total amount steviol glycosides in the composition.

The composition can optionally be expressed in terms of amounts of other known steviol glycosides that are present in lesser amounts. For example, a composition can include one or more of SG201-204 optionally in combination with Reb A, Reb B, and/or stevioside in an amount of about 1 % (wt) or less, about 0.5 % (wt) or less, or about 0.25 % (wt) or less, of the total amount steviol glycosides in the composition. In some embodiments, the amount of SG201-204 in a sweetener composition or beverage can be expressed as a ratio of the total concentration of SG201, SG202, SG203, and SG204 divided by the total concentration of Reb D and Reb. For example, a composition containing 75 wt% of the total glycosides of SG201 and 25% of the total glycosides of Reb M would have a ratio of 3, while a composition containing 1% of the total glycosides of SG202 and 99% of the total glycosides of Reb D + Reb M would have a ratio of about 0.01. In some embodiments, the composition may include a ratio of any one of SG201-204 to the combination of Reb D and Reb M in the range of 0.0001 to 3.5, 0.01 to 3, 0.01 to 2, 0.01 to 1, 0.01 to 0.75, 0.01 to 0.5, 0.1 to 1, 0.5 to 1, 0.05 to 0.5, 1 to 1.5, 1 to 2, 1 to 2.5, 0.00001 to 1, 0.001 to 1, 0.0001 to 0.5, 0.0001 to 0.4, 0.0001 to 0.3, 0.0001 to 0.2, 0.0001 to 0.1, 0.001 to 0.2, 0.001 to 0.01, or 0.001 to 0.1. In some embodiments, the composition may include a ratio of the combination of SG201-204 to the combination of Reb D and Reb M in the range of 0.0001 to 3.5, 0.01 to 3, 0.01 to 2, 0.01 to 1, 0.01 to 0.75, 0.01 to 0.5, 0.1 to 1, 0.5 to 1, 0.05 to 0.5, 1 to 1.5, 1 to 2, 1 to 2.5, 0.00001 to 1, 0.001 to 1, 0.0001 to 0.5, 0.0001 to 0.4, 0.0001 to 0.3, 0.0001 to 0.2, 0.0001 to 0.1, 0.001 to 0.2, 0.001 to 0.01, or 0.001 to 0.1.

The composition can optionally be expressed in terms of the concentration of one or more steviol glycoside(s). Compound(s) SG201, 202, 203, and/or 204, may improve solubility of steviol glycosides in an aqueous solution, and therefore compositions can be prepared having a greater concentration of steviol glycosides in solution. As used herein "instantaneous solubility" refers to the solubility of a steviol glycoside, or mixture of steviol glycosides, that are vigorously mixed with deionized water at room temperature (25°C). As used herein "equilibrium solubility" refers to the solubility of a steviol glycoside, or mixture of steviol glycosides, that is vigorously mixed with deionized water at 80°C for 15 minutes, cooled to room temperature (25°C), and then observed up to 4 days. Clear solutions without precipitates are considered soluble. Unless indicated otherwise herein, the term "solubility" refers to "equilibrium solubility."

In some modes of practice, one or more of compound(s) SG201-204, can be enriched in a composition. The term "enriched" refers to an increase in the amount of compound SG201-204, relative to one or more other compounds that are present in a composition. For example, one or more of compound SG201-204, can be enriched from a fermentation media in which the compounds were produced. In modes of practice, compound SG201-204, can be enriched by the reduction or elimination of components that are not steviol glycosides from the fermentation composition, such as by using enrichment methods as described herein. A composition that is enriched for one or more of compound SG201-204 can be combined with another steviol glycoside composition to improve solubility of those steviol glycosides that are not compounds SG201-204.

In other modes of practice, one or more of compound(s) SG201-204, can be enriched in a composition relative to other steviol glycosides. For example, a composition of steviol glycosides can be enriched to increase the amount(s) of one or more of compound SG201-204 relative to one or more other steviol glycosides in the composition. One or more of compound SG201-204 may be enriched on the basis of their molecular weight, which can be higher than other steviol glycosides, such as Reb D and Reb M.

In exemplary modes of practice, high pressure liquid chromatography is used to prepare a steviol glycoside composition that is enriched for one or more of compound(s) SG201-204 relative to other steviol glycosides in comparison to the amounts of steviol glycosides produced during fermentation. For example, a steviol glycoside composition can include one or more of compound SG201-204 in an amount greater than 6%, greater than about 8%, greater than about 10 %, greater than about 15 %, greater than about 20 %, greater than about 20 %, greater than about 30 %, greater than about 35 %, greater than about 40 %, greater than about 45 %, greater than about 50 %, greater than about 55 %, greater than about 60 %, greater than about 65 %, greater than about 70 %, greater than about 75 %, greater than about 80 %, greater than about 85 %, greater than about 90 %, greater than about 95 %, or greater than 99% relative to the total amount of steviol glycosides in the composition.

For example, following an enrichment process, the steviol glycoside composition can have a combined amount of compounds SG201-204 in the range of about 10 to 30 %, 0.1 to 5%, 2 to 10%, 5 to 20%, 10 to 20% or 15% to 25% and a combined amount of other steviol glycosides, such as Reb D and Reb M in the range of about 70 to 90 %, 75% to 99%, 70% to 95%, 75% to 85%, 80% to 95%, or 85% to 90%.

In yet other modes of practice, one or more of compounds SG201-204 are purified from other steviol glycosides to provide a composition comprising one or more of compounds SG201-204 essentially free of other components (i.e., essentially free of other steviol glycoside and non-steviol glycoside compounds). Such a purified composition can be useful as an additive to other steviol glycoside composition(s), such as to increase the aqueous solubility of the other steviol glycosides to form a composition with higher steviol glycoside concentration. In some embodiments, such a purified composition can be used alone as the only steviol glycoside(s) in a sweetener composition or sweetened composition. In some embodiments, any one of SG201-204 can be used alone as the only steviol glycoside in a sweetener composition or sweetened composition.

Accordingly, other embodiments provide a method of enhancing the solubility of a steviol glycoside in an aqueous composition comprising a step of providing an aqueous composition comprising first and second steviol glycosides, wherein the second steviol glycoside is selected from the group consisting of Reb A, Reb B, Reb M, Reb D, Reb I, Reb Q, Reb N, and stevioside. For example, in the step of providing, the first steviol glycoside can be produced along with the second steviol glycoside, such as when the first and second steviol glycosides are prepared by an enzymatic process (e.g., within a cell, or in a cell-free system).

One or more of compounds SG201-204 can be purified using with preparative liquid chromatography, such as high pressure liquid chromatography (HPLC) or ultra-high pressure liquid chromatography (UHPLC). A steviol glycoside composition with compounds SG201-204 can be dissolved in a mobile phase, such as a mixture of water and an alcohol (e.g., methanol) at a desired ratio (e.g., 60% water, 40% methanol, v/v). The composition can also be heated to enhance dissolution of the steviol glycoside material, such as heating at about 50°C. The solution can also be filtered prior to injection into the column, such as using a 0.2 µm filter. Phenomenex Kinetex XB-C18 5 µm, core-shell silica solid support, and stationary phase of C18 with iso-butyl side chains and TMS endcapping. The flow rate through the column can be based on column properties (such as about 20 mL/min), with a maximum pressure of 400 bar. Compounds SG201-204 can be identified by their elution times from the column. In exemplary flow conditions Compounds 1-4 can elute from the column within 60 minutes. One of skill in the art will appreciate that the elution times for one or more of compounds SG201-204 can vary with changes in solvent and/or equipment. Those experienced in art will also understand that although the process described below assumes certain order of the described steps, this order can be altered in some cases.

Sweetener compositions (also referred to as sweetening compositions), as used herein, refers to compositions that include one or more steviol glycosides, including one or more of compounds SG201-204. For example, a sweetener composition can include one or more of compound(s) SG201-204 along with another steviol glycoside such as Reb M and/or Reb D. If multiple steviol glycosides are present in the sweetener compositions, in some embodiments one or more of compounds SG201-204 can be present in lower amounts in the composition (e.g., less than about 25%, less than about 20%, less than about 15%, or less than about 10%), of the total amount of steviol glycosides in the composition. One or more other steviol glycoside(s) such as Reb M and/or Reb D can be present in a major amount in the composition, such as greater than about 75%, greater than about 80%, greater than about 85%, greater than about 90%, or greater than about 95%, of the total amount of steviol glycosides in the composition.

In one embodiment, one or more compounds of SG201-204, are present in a sweetener composition at about 0.05 to 70 wt% of the total content of the sweetener composition, e.g., about 0.1 to 50, 0.5 to 70, 1 to 50, 1 to 35, 2 to 25, 3 to 20, 5 to 15, 0.1 to 15, 0.5 to 10, 1 to 5%, etc. In one embodiment, steviol glycosides other than Reb D, Reb M, Reb G, Reb O, Reb N, and/or Reb E, including for example one or more compounds SG201-204, are at a weight ratio of the total of all other glycosides of 1:1 to 1:20, 1:1.5 to 1:15, 1:2 to 1:10, 1:2.5 to 1:7.5, or 1:3 to 1:5.

The sweetener composition can optionally include another sweetener, an additive, a liquid carrier, or combinations thereof. Sweetener compositions are used to sweeten other compositions (sweetenable compositions) such as foods, beverages, medicines, oral hygiene compositions, nutraceuticals, and the like.

Sweetenable compositions, as used herein, mean substances which are contacted with the mouth of man or animal, including substances which are taken into but subsequently ejected from the mouth (such as a mouthwash rinse) and substances which are drunk, eaten, swallowed or otherwise ingested, and are suitable for human or animal consumption when used in a generally acceptable range. Sweetenable compositions are precursor compositions to sweetened compositions and are converted to sweetened compositions by combining the sweetenable compositions with at least one sweetening composition and optionally one or more other sweetenable compositions and/or other ingredients.

Sweetened compositions, as used herein, mean substances that are derived from constituents including at least one sweetenable composition and at least one sweetener composition. In some modes of practice, a sweetened composition may be used itself as a sweetening composition to sweeten still yet further sweetenable compositions. In some modes of practice, a sweetened composition may be used as a sweetenable composition that is further sweetened with one or more additional sweetening compositions. For example, a beverage with no sweetener component is a type of sweetenable composition. A sweetener composition comprising at least one of compounds SG201-204, optionally along with another steviol glycoside, such as Reb M and/or Reb D, can be added to the un-sweetened beverage, thereby providing a sweetened beverage. The sweetened beverage is a type of sweetened composition.

In some preparations, steviol glycosides, including one or more of compounds SG201-204, provide the sole sweetener component in a sweetening composition.

In some embodiments, a sweetening composition comprises steviol glycosides, including one or more of compounds SG201-204, in an amount effective to provide a sweetness strength equivalent to a specified amount of sucrose. The amount of sucrose in a reference solution may be described in degrees Brix (°Bx). One degree Brix is 1 gram of sucrose in 100 grams of solution and represents the strength of the solution as percentage by weight (% w/w). For example, a sweetener composition contains one or more steviol glycosides, including one or more of compounds SG201-204, in an amount effective to provide a sweetness equivalent from about 0.50 to 14 degrees Brix of sugar when present in a sweetened composition, such as, for example, from about 5 to about 11 degrees Brix, from about 4 to about 7 degrees Brix, or about 5 degrees Brix.

The amount of steviol glycosides in the sweetener composition may vary. Steviol glycosides, including one or more of compounds SG201-204, can be present in a sweetener composition in any amount to impart the desired sweetness when the sweetener composition is incorporated into a sweetened composition. For example, Reb M and/or Reb D, along with one or more of compounds SG201-204, are present in the sweetener composition in an amount effective to provide total steviol glycoside concentration from about 1 ppm to about 10,000 ppm when present in a sweetened composition, In another embodiment, the steviol glycosides are present in the sweetener composition in an amount effective to provide a steviol glycoside concentration in the range of about 10 ppm to about 1,000 ppm, more specifically about 10 ppm to about 800 ppm, about 50 ppm to about 800 ppm, about 50 ppm to about 600 ppm, or about 200 ppm to about 500 ppm. In one embodiment, steviol glycosides other than Reb D, Reb M, Reb G, Reb O, Reb N, and/or Reb E, or other than Reb M, Reb D, Reb B and Reb A, or other than Reb D and Reb M, such as one or more of compounds SG201-204, are present in a beverage at about at least 1 ppm to about 600 ppm, e.g., about 50 ppm to about 500 ppm, including at least 1, 5, 10, 20, 30, 40, 50, 125, 150, 150, 175, or 200 ppm. Unless otherwise expressly stated, ppm is on a weight basis.

In some embodiments, a sweetener composition having the steviol glycosides, including one or more of compounds SG201-204, also contain one or more additional non-steviol glycoside sweetener compound(s). The non-steviol glycoside sweetener compounds can be any type of sweetener, for example, a sweetener obtained from a plant or plant product, or a physically or chemically modified sweetener obtained from a plant, or a synthetic sweetener.

For example, exemplary non-steviol glycoside sweeteners include sucrose, fructose, glucose, erythritol, maltitol, lactitol, sorbitol, mannitol, xylitol, tagatose, trehalose, galactose, rhamnose, cyclodextrin (e.g., a-cyclodextrin, β-cyclodextrin, and γ-cyclodextrin), ribulose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, glucosamine, mannosamine, fucose, fuculose, glucuronic acid, gluconic acid, glucono-lactone, abequose, galactosamine, xylo-oligosaccharides (xylotriose, xylobiose and the like), gentio-oligoscaccharides (gentiobiose, gentiotriose, gentiotetraose and the like), galacto-oligosaccharides, sorbose, ketotriose (dehydroxyacetone), aldotriose (glyceraldehyde), nigero-oligosaccharides, fructooligosaccharides (kestose, nystose and the like), maltotetraose, maltotriol, tetrasaccharides, mannan-oligosaccharides, malto-oligosaccharides (maltotriose, maltotetraose, maltopentaose, maltohexaose, maltoheptaose and the like), dextrins, lactulose, melibiose, raffinose, rhamnose, ribose, isomerized liquid sugars such as high fructose corn/starch syrup (HFCS/HFSS) (e.g., HFCS55, HFCS42, or HFCS90), coupling sugars, soybean oligosaccharides, glucose syrup and combinations thereof. D- or L-configurations can be used when applicable.

The steviol glycosides (including one or more of compounds SG201-204), and carbohydrate sweetener may be present in any weight ratio, such as, for example, from about 1:14,000 to about 100: 1, such as, for example, about 1:100. Carbohydrates are present in the sweetener composition in an amount effective to provide a concentration from about 100 ppm to about 140,000 ppm when present in a sweetened composition, such as, for example, a beverage.

In other embodiments, the sweetener composition including the steviol glycosides (including one or more of compounds SG201-204), additionally include one or more synthetic sweeteners. In one embodiment, a synthetic has a sweetness potency greater than sucrose, fructose, and/or glucose, yet has less calories than sucrose, fructose, and/or glucose. Exemplary synthetic non-steviol glycoside sweeteners include sucralose, potassium acesulfame, acesulfame acid and salts thereof, aspartame, alitame, saccharin and salts thereof, neohesperidin dihydrochalcone, cyclamate, cyclamic acid and salts thereof, neotame, advantame, glucosylated steviol glycosides (GSGs) and combinations thereof. In embodiments where the sweetener composition includes the steviol glycosides (including one or more of compounds SG201-204) and synthetic sweetener, the synthetic sweetener can be present in an amount effective to provide a concentration from about 0.3 ppm to about 3,500 ppm when present in a sweetened composition, such as, for example, a beverage.

The sweetener compositions can be customized to provide a desired calorie content. For example, sweetener compositions can be "full-calorie", such that they impart the desired sweetness when added to a sweetenable composition (such as, for example, a beverage) and have about 120 calories per 8 oz serving. Alternatively, sweetener compositions can be "mid-calorie", such that they impart the desired sweetness when added to a sweetenable composition (such as, for example, as beverage) and have less than about 60 calories per 8 oz serving. In other embodiments, sweetener compositions can be "low-calorie", such that they impart the desired sweetness when added to a sweetenable composition (such as, for example, as beverage) and have less than 40 calories per 8 oz serving. In still other embodiments, the sweetener compositions can be "zero-calorie", such that they impart the desired sweetness when added to a sweetenable composition (such as, for example, a beverage) and have less than 5 calories per 8 oz. serving. Non-calorie compositions are "non-nutritive." In some embodiments, low calorie compositions can also be referred to as "non-nutritive."

The weight ratio of the total amount of sweetener compositions used to sweeten a sweetened composition can vary over a wide range. In many embodiments, this weight ratio is in the range from 1:10,000 to 10:1.

In addition to the steviol glycosides (including one or more of compounds SG201-204) the sweetener compositions can optionally include a liquid carrier, binder matrix, additional additives, and/or the like. In some embodiments, the sweetener composition contains additives including, but not limited to, carbohydrates, polyols, amino acids and their corresponding salts, poly- amino acids and their corresponding salts, sugar acids and their corresponding salts, nucleotides, organic acids, inorganic acids, organic salts including organic acid salts and organic base salts, inorganic salts, bitter compounds, flavorants and flavoring ingredients, astringent compounds, proteins or protein hydrolysates, surfactants, emulsifiers, weighing agents, gums, antioxidants, colorants, flavonoids, alcohols, polymers and combinations thereof. In some embodiments, the additives act to improve the temporal and flavor profile of the sweetener to provide a sweetener composition with a favorable taste, such as a taste similar to sucrose.

In one embodiment, the sweetener compositions with steviol glycosides (including one or more of compounds SG201-204) contain one or more polyols. The term "polyol", as used herein, refers to a molecule that contains more than one hydroxyl group. In some embodiments, a polyol may be a diol, triol, or a tetraol which contains 2, 3, and 4 hydroxyl groups respectively. A polyol also may contain more than 4 hydroxyl groups, such as a pentaol, hexaol, heptaol, or the like, which contain 5, 6, 7, or even more hydroxyl groups, respectively. Additionally, a polyol also may be a sugar alcohol, polyhydric alcohol, polymer comprising OH functionality, or polyalcohol which is a reduced form of a carbohydrate, wherein a carbonyl group (aldehyde or ketone, reducing sugar) has been reduced to a primary or secondary hydroxyl group.

Exemplary polyols include erythritol, maltitol, mannitol, sorbitol, lactitol, xylitol, isomalt, propylene glycol, glycerol (glycerin), threitol, galactitol, palatinose, reduced isomalto-oligosaccharides, reduced xylo-oligosaccharides, reduced gentio-oligosaccharides, reduced maltose syrup, reduced glucose syrup, and sugar alcohols or any other carbohydrates capable of being reduced which do not adversely affect the taste of the sweetener composition.

Exemplary amounts of polyol provide a concentration in the range of about 100 ppm to about 250,000 ppm when present in a sweetened composition, more specifically about 400 ppm to about 80,000 ppm, or about 5,000 ppm to about 40,000 ppm, based on the total weight of the sweetened composition.

Exemplary amino acid additives include any compound comprising at least one amino functionality and at least one acid functionality. Examples include, but are not limited to, aspartic acid, arginine, glycine, glutamic acid, proline, threonine, theanine, cysteine, cystine, alanine, valine, tyrosine, leucine, arabinose, trans-4-hydroxyproline, isoleucine, asparagine, serine, lysine, histidine, ornithine, methionine, carnitine, aminobutyric acid (α-, β-, and/or δ-isomers), glutamine, hydroxyproline, taurine, norvaline, sarcosine, and their salt forms such as sodium or potassium salts or acid salts.

Exemplary amounts of amino acid provide a concentration in the range of about 10 ppm to about 50,000 ppm, or more specifically about 1,000 ppm to about 10,000 ppm, about 2,500 ppm to about 5,000 ppm, or about 250 ppm to about 7,500 ppm, based on the total weight of the sweetened composition.

Exemplary sugar acid additives include, but are not limited to, aldonic, uronic, aldaric, alginic, gluconic, glucuronic, glucaric, galactaric, galacturonic, and salts thereof (e.g., sodium, potassium, calcium, magnesium salts or other physiologically acceptable salts), and combinations thereof.

Exemplary nucleotide additives include, but are not limited to, inosine monophosphate ("IMP"), guanosine monophosphate ("GMP"), adenosine monophosphate ("AMP"), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, alkali or alkaline earth metal salts thereof, and combinations thereof. The nucleotides described herein also may comprise nucleotide-related additives, such as nucleosides or nucleic acid bases (e.g., guanine, cytosine, adenine, thymine, uracil). In some embodiments, a nucleotide can be present in the sweetener composition to provide a concentration in the range of about 5 ppm to about 1,000 ppm based on the total weight of the sweetened composition.

Exemplary organic acid additives include any compound which comprises a - COOH moiety, such as, for example, C2-C30 carboxylic acids, substituted hydroxyl C2-C30 carboxylic acids, butyric acid (ethyl esters), substituted butyric acid (ethyl esters), benzoic acid, substituted benzoic acids (e.g., 2,4-dihydroxybenzoic acid), substituted cinnamic acids, hydroxyacids, substituted hydroxybenzoic acids, anisic acid substituted cyclohexyl carboxylic acids, tannic acid, aconitic acid, lactic acid, tartaric acid, citric acid, isocitric acid, gluconic acid, glucoheptonic acids, adipic acid, hydroxycitric acid, malic acid, fruitaric acid (a blend of malic, fumaric, and tartaric acids), fumaric acid, maleic acid, succinic acid, chlorogenic acid, salicylic acid, creatine, caffeic acid, bile acids, acetic acid, ascorbic acid, alginic acid, erythorbic acid, polyglutamic acid, glucono delta lactone, and their alkali or alkaline earth metal salt derivatives thereof. In addition, the organic acid additives also may be in either the D- or L-configuration. Salts of organic acids are also contemplated. In exemplary embodiments, an organic acid or salt thereof is present in the sweetener composition in an amount from about 10 ppm to about 5,000 ppm, based on the total weight of the sweetener composition.

Exemplary inorganic acid additives include, but are not limited to, phosphoric acid, phosphorous acid, polyphosphoric acid, hydrochloric acid, sulfuric acid, carbonic acid, sodium dihydrogen phosphate, and alkali or alkaline earth metal salts thereof (e.g., inositol hexaphosphate Mg/Ca).

Exemplary bitter compound additives include, but are not limited to, caffeine, quinine, urea, bitter orange oil, naringin, quassia, and salts thereof.

Exemplary flavorant and flavoring ingredient additives, but are not limited to, vanillin, vanilla extract, mango extract, cinnamon, citrus, coconut, ginger, viridiflorol, almond, menthol (including menthol without mint), grape skin extract, and grape seed extract. In some embodiments, a flavorant is present in the sweetener composition in an amount effective to provide a concentration from about 0.1 ppm to about 4,000 ppm when present in a sweetened composition, such as, for example, a beverage, based on the total weight of the sweetened composition.

Exemplary polymer additives include, chitosan, pectin, pectic, pectinic, polyuronic, polygalacturonic acid, starch, food hydrocolloid or crude extracts thereof (e.g., gum acacia Senegal (Fibergum^{™}), gum acacia seyal, carageenan), poly-L-lysine (e.g., poly-L-α-lysine or poly-L-e-lysine), poly-L-ornithine (e.g., poly-L- α-ornithine or poly-L-e-ornithine), polypropylene glycol, polyethylene glycol, poly(ethylene glycol methyl ether), polyarginine, polyaspartic acid, polyglutamic acid, polyethylene imine, alginic acid, sodium alginate, propylene glycol alginate, and sodium polyethyleneglycolalginate, sodium hexametaphosphate and its salts, and other cationic polymers and anionic polymers. In some embodiments, a polymer additive is present in the sweetener composition in an amount effective to provide a concentration from about 30 ppm to about 2,000 ppm when present in a sweetened composition, such as, for example, a beverage, based on the total weight of the sweetened composition.

Exemplary protein or protein hydrolysate additives include, but are not limited to, bovine serum albumin (BSA), whey protein, soluble rice protein, soy protein, protein isolates, protein hydrolysates, reaction products of protein hydrolysates, glycoproteins, and/or proteoglycans containing amino acids, collagen (e.g., gelatin), partially hydrolyzed collagen (e.g., hydrolyzed fish collagen), and collagen hydrolysates (e.g., porcine collagen hydrolysate). In some embodiments, a protein hydrosylate is present in the sweetener composition in an amount effective to provide a concentration from about 200 ppm to about 50,000 ppm when present in a sweetened composition, such as, for example, a beverage, based on the total weight of the sweetened composition.

Exemplary surfactant additives include, but are not limited to, polysorbates (e.g., polyoxyethylene sorbitan monooleate (polysorbate 80), polysorbate 20, polysorbate 60), sodium dodecylbenzenesulfonate, dioctyl sulfosuccinate or dioctyl sulfosuccinate sodium, sodium dodecyl sulfate, cetylpyridinium chloride (hexadecylpyridinium chloride), hexadecyltrimethylammonium bromide, sodium cholate, carbamoyl, choline chloride, sodium glycocholate, sodium taurodeoxycholate, lauric arginate, sodium stearoyl lactylate, sodium taurocholate, lecithins, sucrose oleate esters, sucrose stearate esters, sucrose palmitate esters, sucrose laurate esters, and other emulsifiers, and the like. In some embodiments, a surfactant additive is present in the sweetener composition in an amount effective to provide a concentration from about 30 ppm to about 2,000 ppm when present in a sweetened composition, such as, for example, a beverage, based on the total weight of the sweetened composition.

Exemplary flavonoid additives are classified as flavonols, flavones, flavanones, flavan-3-ols, isoflavones, or anthocyanidins. Non-limiting examples of flavonoid additives include, but are not limited to, catechins (e.g., green tea extracts such as Polyphenon^{™} 60, Polyphenon^{™} 30, and Polyphenon^{™} 25 (Mitsui Norin Co., Ltd., Japan), polyphenols, rutins (e.g., enzyme modified rutin Sanmelin^{™} AO (San-fi Gen F.F.I., Inc., Osaka, Japan)), neohesperidin, naringin, neohesperidin dihydrochalcone, and the like. In some embodiments, a flavonoid additive is present in the sweetener composition in an amount effective to provide a concentration from about 0.1 ppm to about 1,000 ppm when present in sweetened composition, such as, for example, a beverage, based on the total weight of the sweetened composition.

Exemplary alcohol additives include, but are not limited to, ethanol. In some embodiments, an alcohol additive is present in the sweetener composition in an amount effective to provide a concentration from about 625 ppm to about 10,000 ppm when present in a sweetened composition, such as, for example, a beverage, based on the total weight of the sweetened composition.

The sweetener composition can also contain one or more functional ingredients, which provide a real or perceived heath benefit to the composition. Functional ingredients include, but are not limited to, saponins, antioxidants, dietary fiber sources, fatty acids, vitamins, glucosamine, minerals, preservatives, hydration agents, probiotics, prebiotics, weight management agents, osteoporosis management agents, phytoestrogens, long chain primary aliphatic saturated alcohols, phytosterols and combinations thereof.

Saponins are glycosidic plant products comprising an aglycone ring structure and one or more sugar moieties. The combination of the nonpolar aglycone and the water soluble sugar moiety gives saponins surfactant properties, which allow them to form a foam when shaken in an aqueous solution.

As used herein "antioxidant" refers to any substance which inhibits, suppresses, or reduces oxidative damage to cells and biomolecules. Without being bound by theory, it is believed that antioxidants inhibit, suppress, or reduce oxidative damage to cells or biomolecules by stabilizing free radicals before they can cause harmful reactions. As such, antioxidants may prevent or postpone the onset of some degenerative diseases.

Examples of suitable antioxidants of this disclosure include, but are not limited to, vitamins, vitamin cofactors, minerals, hormones, carotenoids, carotenoid terpenoids, non-carotenoid terpenoids, flavonoids, flavonoid polyphenolics (e.g., bioflavonoids), flavonols, flavones, phenols, polyphenols, esters of phenols, esters of polyphenols, nonflavonoid phenolics, isothiocyanates, and combinations thereof. In some embodiments, the antioxidant is vitamin A, vitamin C, vitamin E, ubiquinone, mineral selenium, manganese, melatonin, a-carotene, β-carotene, lycopene, lutein, zeanthin, crypoxanthin, reservatol, eugenol, quercetin, catechin, gossypol, hesperetin, curcumin, ferulic acid, thymol, hydroxytyrosol, tumeric, thyme, olive oil, lipoic acid, glutathinone, gutamine, oxalic acid, tocopherol-derived compounds, butylated hydroxyanisole (BHA), butylated hydroxytoluene (BHT), ethylenediaminetetraacetic acid (EDTA), tert-butylhydroquinone, acetic acid, pectin, tocotrienol, tocopherol, coenzyme Q10, zeaxanthin, astaxanthin, canthaxantin, saponins, limonoids, kaempfedrol, myricetin, isorhamnetin, proanthocyanidins, quercetin, rutin, luteolin, apigenin, tangeritin, hesperetin, naringenin, erodictyol, flavan- 3-ols (e.g., anthocyanidins), gallocatechins, epicatechin and its gallate forms, epigallocatechin and its gallate forms (ECGC) theaflavin and its gallate forms, thearubigins, isoflavone phytoestrogens, genistein, daidzein, glycitein, anythocyanins, cyaniding, delphinidin, malvidin, pelargonidin, peonidin, petunidin, ellagic acid, gallic acid, salicylic acid, rosmarinic acid, cinnamic acid and its derivatives (e.g., ferulic acid), chlorogenic acid, chicoric acid, gallotannins, ellagitannins, anthoxanthins, betacyanins and other plant pigments, silymarin, citric acid, lignan, antinutrients, bilirubin, uric acid, R-a-lipoic acid, N-acetylcysteine, emblicanin, apple extract, apple skin extract (applephenon), rooibos extract red, rooibos extract, green, hawthorn berry extract, red raspberry extract, green coffee antioxidant (GCA), aronia extract 20%, grape seed extract (VinOseed), cocoa extract, hops extract, mangosteen extract, mangosteen hull extract, cranberry extract, pomegranate extract, pomegranate hull extract, pomegranate seed extract, hawthorn berry extract, pomella pomegranate extract, cinnamon bark extract, grape skin extract, bilberry extract, pine bark extract, pycnogenol, elderberry extract, mulberry root extract, wolf erry (gogi) extract, blackberry extract, blueberry extract, blueberry leaf extract, raspberry extract, turmeric extract, citrus bioflavonoids, black currant, ginger, acai powder, green coffee bean extract, green tea extract, and phytic acid, or combinations thereof. In alternate embodiments, the antioxidant is a synthetic antioxidant such as butylated hydroxytolune or butylated hydroxyanisole, for example. Other sources of suitable antioxidants of this disclosure include, but are not limited to, fruits, vegetables, tea, cocoa, chocolate, spices, herbs, rice, organ meats from livestock, yeast, whole grains, or cereal grains.

Particular antioxidants belong to the class of phytonutrients called polyphenols (also known as "polyphenolics"), which are a group of chemical substances found in plants, characterized by the presence of more than one phenol group per molecule. A variety of health benefits may be derived from polyphenols, including prevention of cancer, heart disease, and chronic inflammatory disease and improved mental strength and physical strength, for example. Suitable polyphenols include but are not limited to catechins, proanthocyanidins, procyanidins, anthocyanins, quercerin, rutin, reservatrol, isoflavones, curcumin, punicalagin, ellagitannin, hesperidin, naringin, citrus flavonoids, chlorogenic acid, other similar materials, and combinations thereof.

Numerous polymeric carbohydrates having significantly different structures in both composition and linkages fall within the definition of dietary fiber. Such compounds are well known to those skilled in the art, non-limiting examples of which include non-starch polysaccharides, lignin, cellulose, methylcellulose, the hemicelluloses, β-glucans, pectins, gums, mucilage, waxes, inulins, oligosaccharides, fructooligosaccharides, cyclodextrins, chitins, and combinations thereof.

As used herein, "fatty acid" refers to any straight chain monocarboxylic acid and includes saturated fatty acids, unsaturated fatty acids, long chain fatty acids, medium chain fatty acids, short chain fatty acids, fatty acid precursors (including omega-9 fatty acid precursors), and esterified fatty acids. As used herein, "long chain polyunsaturated fatty acid" refers to any polyunsaturated carboxylic acid or organic acid with a long aliphatic tail. As used herein, "omega-3 fatty acid" refers to any polyunsaturated fatty acid having a first double bond as the third carbon-carbon bond from the terminal methyl end of its carbon chain. In particular embodiments, the omega-3 fatty acid may comprise a long chain omega-3 fatty acid. As used herein, "omega-6 fatty acid" refers to any polyunsaturated fatty acid having a first double bond as the sixth carbon-carbon bond from the terminal methyl end of its carbon chain.

As used herein, the at least one vitamin may be single vitamin or a plurality of vitamins as a functional ingredient for the sweetener and sweetened compositions provided herein. Generally, according to particular embodiments of this disclosure, the at least one vitamin is present in the sweetener composition or sweetened composition in an amount sufficient to promote health and wellness.

Vitamins are organic compounds that the human body needs in small quantities for normal functioning. The body uses vitamins without breaking them down, unlike other nutrients such as carbohydrates and proteins. To date, thirteen vitamins have been recognized, and one or more can be used in the functional sweetener and sweetened compositions herein. Suitable vitamins include, vitamin A, vitamin D, vitamin E, vitamin K, vitamin Bl, vitamin B2, vitamin B3, vitamin B5, vitamin B6, vitamin B7, vitamin B9, vitamin B 12, and vitamin C. Many of vitamins also have alternative chemical names, non-limiting examples of which are provided below.

In certain embodiments, the functional ingredient comprises glucosamine or chondroitin sulfate. Glucosamine, also called chitosamine, is an amino sugar that is believed to be an important precursor in the biochemical synthesis of glycosylated proteins and lipids. D-glucosamine occurs in the cartilage in the form of glucosamine-6- phosphate, which is synthesized from fructose-6-phosphate and glutamine. However, glucosamine also is available in other forms, non-limiting examples of which include glucosamine hydrochloride, glucosamine sulfate, N-acetyl-glucosamine, or any other salt forms or combinations thereof.

In certain embodiments, the functional ingredient comprises at least one mineral. Minerals comprise inorganic chemical elements required by living organisms. Minerals are comprised of a broad range of compositions (e.g., elements, simple salts, and complex silicates) and also vary broadly in crystalline structure. They may naturally occur in foods and beverages, may be added as a supplement, or may be consumed or administered separately from foods or beverages. In particular embodiments of this disclosure, the mineral is chosen from bulk minerals, trace minerals or combinations thereof. Non-limiting examples of bulk minerals include calcium, chlorine, magnesium, phosphorous, potassium, sodium, and sulfur. Non-limiting examples of trace minerals include chromium, cobalt, copper, fluorine, iron, manganese, molybdenum, selenium, zinc, and iodine. Although iodine generally is classified as a trace mineral, it is required in larger quantities than other trace minerals and often is categorized as a bulk mineral.

In certain embodiments, the functional ingredient comprises at least one preservative. In particular embodiments of this disclosure, the preservative is chosen from antimicrobials, antioxidants, antienzymatics or combinations thereof. Non-limiting examples of antimicrobials include sulfites, propionates, benzoates, sorbates, nitrates, nitrites, bacteriocins, salts, sugars, acetic acid, dimethyl dicarbonate (DMDC), ethanol, and ozone.

In certain embodiments, the functional ingredient is at least one hydration agent. Hydration products help the body to replace fluids that are lost through excretion. In a particular embodiment, the hydration product is a composition that helps the body to replace fluids that are lost during exercise. Accordingly, in a particular embodiment, the hydration product is an electrolyte, non-limiting examples of which include sodium, potassium, calcium, magnesium, chloride, phosphate, bicarbonate, and combinations thereof. In particular embodiments of this disclosure, the hydration product is a carbohydrate to supplement energy stores burned by muscles. In another particular embodiment, the hydration agent is at least one flavanol that provides cellular rehydration. Flavanols are a class of substances present in plants, and generally comprise a 2-phenylbenzopyrone molecular skeleton attached to one or more chemical moieties. In a particular embodiment, the hydration agent comprises a glycerol solution to enhance exercise endurance. The ingestion of a glycerol containing solution has been shown to provide beneficial physiological effects, such as expanded blood volume, lower heart rate, and lower rectal temperature.

In certain embodiments, the functional ingredient comprises at least one probiotic, prebiotic and combination thereof. Probiotics comprise microorganisms that benefit health when consumed in an effective amount. Desirably, probiotics beneficially affect the human body's gastrointestinal microflora and impart health benefits apart from nutrition. Probiotics may include, without limitation, bacteria, yeasts, and fungi. Examples of probiotics include, but are not limited to, bacteria of the genus Lactobacilli, Bifidobacteria, Streptococci, or combinations thereof, that confer beneficial effects to humans. Prebiotics are compositions that promote the growth of beneficial bacteria in the intestines.

In certain embodiments, the functional ingredient is at least one weight management agent. As used herein, "a weight management agent" includes an appetite suppressant and/or a thermogenesis agent. As used herein, the phrases "appetite suppressant", "appetite satiation compositions", "satiety agents", and "satiety ingredients" are synonymous. The phrase "appetite suppressant" describes macronutrients, herbal extracts, exogenous hormones, anorectics, anorexigenics, pharmaceutical drugs, and combinations thereof, that when delivered in an effective amount, suppresses, inhibits, reduces, or otherwise curtails a person's appetite. The phrase "thermogenesis agent" describes macronutrients, herbal extracts, exogenous hormones, anorectics, anorexigenics, pharmaceutical drugs, and combinations thereof, that when delivered in an effective amount, activate or otherwise enhance a person's thermogenesis or metabolism.

In certain embodiments, the functional ingredient is at least one osteoporosis management agent. In certain embodiments, the osteoporosis management agent is at least one calcium source. According to a particular embodiment, the calcium source is any compound containing calcium, including salt complexes, solubilized species, and other forms of calcium. According to a particular embodiment, the osteoporosis management agent is a magnesium source. The magnesium source is any compound containing magnesium, including salt complexes, solubilized species, and other forms of magnesium. In other embodiments, the osteoporosis agent is chosen from vitamins D, C, K, their precursors and/or beta-carotene and combinations thereof.

In certain embodiments, the functional ingredient is at least one phytoestrogen. In one embodiment, a sweetener composition comprises at least one phytoestrogen. As used herein, "phytoestrogen" refers to any substance which, when introduced into a body causes an estrogen-like effect of any degree. Examples of suitable phytoestrogens for embodiments of this disclosure include, but are not limited to, isoflavones, stilbenes, lignans, resorcyclic acid lactones, coumestans, coumestrol, equol, and combinations thereof.

Isoflavones belong to the group of phytonutrients called polyphenols. In general, polyphenols (also known as "polyphenolics"), are a group of chemical substances found in plants, characterized by the presence of more than one phenol group per molecule. Suitable phytoestrogen isoflavones in accordance with embodiments of this disclosure include genistein, daidzein, glycitein, biochanin A, formononetin, their respective glycosides and glycoside conjugates, matairesinol, secoisolariciresinol, enterolactone, enterodiol, textured vegetable protein, and combinations thereof.

In certain embodiments, the functional ingredient is at least one long chain primary aliphatic saturated alcohol. Non-limiting examples of particular long-chain primary aliphatic saturated alcohols for use in particular embodiments of the disclosure include the 8 carbon atom 1-octanol, the 9 carbon 1-nonanol, the 10 carbon atom 1-decanol, the 12 carbon atom 1-dodecanol, the 14 carbon atom 1-tetradecanol, the 16 carbon atom 1 -hexadecanol, the 18 carbon atom 1 -octadecanol, the 20 carbon atom 1-eicosanol, the 22 carbon 1- docosanol, the 24 carbon 1-tetracosanol, the 26 carbon 1-hexacosanol, the 27 carbon 1- heptacosanol, the 28 carbon 1-octanosol, the 29 carbon 1-nonacosanol, the 30 carbon 1- triacontanol, the 32 carbon 1 - dotriacontanol, and the 34 carbon 1 -tetracontanol.

In certain embodiments, the functional ingredient is at least one phytosterol, phytostanol or combination thereof. As used herein, the phrases "stanol", "plant stanol" and "phytostanol" are synonymous. Sterols are a subgroup of steroids with a hydroxyl group at C-3. Generally, phytosterols have a double bond within the steroid nucleus, like cholesterol; however, phytosterols also may comprise a substituted sidechain (R) at C-24, such as an ethyl or methyl group, or an additional double bond. The structures of phytosterols are well known to those of skill in the art. Phytosterols well known to those or ordinary skill in the art include 4-desmethylsterols (e.g., β-sitosterol, campesterol, stigmasterol, brassicasterol, 22-dehydrobrassicasterol, and Δ5- avenasterol), 4-monomethyl sterols, and 4,4-dimethyl sterols (triterpene alcohols) (e.g., cycloartenol, 24-methylenecycloartanol, and cyclobranol). Examples of phytostanols include β-sitostanol, campestanol, cycloartanol, and saturated forms of other triterpene alcohols.

Generally, the amount of functional ingredient in the sweetener composition or sweetened composition varies widely depending on the particular sweetener composition or sweetened composition and the desired functional ingredient. Those of ordinary skill in the art will readily acertain the appropriate amount of functional ingredient for each sweetener composition or sweetened composition.

Steviol glycosides, including compounds SG201-204, or sweetener compositions comprising steviol glycosides, including one or more of compounds SG201-204, can be incorporated in any known edible material (referred to herein as a "sweetenable composition") or other composition intended to be ingested and/or contacted with the mouth of a human or animal, such as, for example, pharmaceutical compositions, edible gel mixes and compositions, dental and oral hygiene compositions, foodstuffs (confections, condiments, chewing gum, cereal compositions, baked goods, baking goods, cooking adjuvants, dairy products, and tabletop sweetener compositions), beverages, and other beverage products (e.g., beverage mixes, beverage concentrates, etc.).

In one embodiment, a sweetened composition is derived from ingredients comprising a sweetenable composition and additionally steviol glycosides, including one or more of compounds SG201-204. In another embodiment, the sweetened composition is derived from ingredients comprising a sweetener composition comprising Steviol glycosides, including one or more of compounds SG201-204,. The sweetened compositions can optionally include one or more additives, liquid carriers, binders, sweeteners, functional ingredients, other adjuvants, and combinations thereof.

In one embodiment, a pharmaceutical composition contains a pharmaceutically active substance (including prodrug forms thereof) and steviol glycosides, including one or more of compounds SG201-204. In another embodiment, a pharmaceutical composition contains a pharmaceutically active substance and a sweetener composition comprising steviol glycosides, including one or more of compounds SG201-204. The steviol glycoside sweetener composition can be present as an excipient material in the pharmaceutical composition, which can mask a bitter or otherwise undesirable taste of a pharmaceutically active substance or another excipient material. The pharmaceutical composition may be in the form of a tablet, a capsule, a liquid, an aerosol, a powder, an effervescent tablet or powder, a syrup, an emulsion, a suspension, a solution, or any other form for providing the pharmaceutical composition to a patient. In particular embodiments, the pharmaceutical composition may be in a form for oral administration, buccal administration, sublingual administration, or any other route of administration as known in the art.

As referred to herein, "pharmaceutically active substance" means any drug, drug formulation, medication, prophylactic agent, therapeutic agent, or other substance having biological activity. Pharmaceutically active substances also include prodrug forms of these. As referred to herein, "excipient material" refers to any other ingredient used in a pharmaceutically active composition used in combination with pharmaceutically active substance(s) that are present

(including prodrugs thereof. Excipients included but are not limited to inactive substances used as a vehicle for an active ingredient, such as any material to facilitate handling, stability, dispersibility, wettability, and/or release kinetics of a pharmaceutically active substance.

Suitable pharmaceutically active substances include, but are not limited to, medications for the gastrointestinal tract or digestive system, for the cardiovascular system, for the central nervous system, for pain or consciousness, for musculo-skeletal disorders, for the eye, for the ear, nose and oropharynx, for the respiratory system, for endocrine problems, for the reproductive system or urinary system, for contraception, for obstetrics and gynecology, for the skin, for infections and infestations, for immunology, for allergic disorders, for nutrition, for neoplastic disorders, for diagnostics, for euthanasia, or other biological functions or disorders.

Examples of suitable pharmaceutically active substances for embodiments of the present disclosure include, but are not limited to, antacids, reflux suppressants, antiflatulents, antidopaminergics, proton pump inhibitors, cytoprotectants, prostaglandin analogues, laxatives, antispasmodics, antidiarrhoeals, bile acid sequestrants, opioids, beta-receptor blockers, calcium channel blockers, diuretics, cardiac glycosides, antiarrhythmics, nitrates, antianginals, vasoconstrictors, vasodilators, peripheral activators, ACE inhibitors, angiotensin receptor blockers, alpha blockers, anticoagulants, heparin, antiplatelet drugs, fibrinolytics, anti-hemophilic factors, haemostatic drugs, hypolipidaemic agents, statins, hynoptics, anaesthetics, antipsychotics, antidepressants, anti-emetics, anticonvulsants, antiepileptics, anxiolytics, barbiturates, movement disorder drugs, stimulants, benzodiazepines, cyclopyrrolones, dopamine antagonists, antihistamines, cholinergics, anticholinergics, emetics, cannabinoids, analgesics, muscle relaxants, antibiotics, aminoglycosides, anti-virals, anti-fungals, anti- inflammatories, anti-gluacoma drugs, sympathomimetics, steroids, ceruminolytics, bronchodilators, NSAIDS, antitussive, mucolytics, decongestants, corticosteroids, androgens, antiandrogens, gonadotropins, growth hormones, insulin, antidiabetics, thyroid hormones, calcitonin, diphosponates, vasopressin analogues, alkalizing agents, quinolones, anticholinesterase, sildenafil, oral contraceptives, Hormone Replacement Therapies, bone regulators, follicle stimulating hormones, luteinizings hormones, gamolenic acid, progestogen, dopamine agonist, oestrogen, prostaglandin, gonadorelin, clomiphene, tamoxifen, diethylstilbestrol, antileprotics, antituberculous drugs, antimalarials, anthelmintics, antiprotozoal, antiserums, vaccines, interferons, tonics, vitamins, cytotoxic drugs, sex hormones, aromatase inhibitors, somatostatin inhibitors, or similar type substances, or combinations thereof. Such components generally are recognized as safe (GRAS) and/or are U.S. Food and Drug Administration (FDA) approved.

The pharmaceutical composition also may comprise other pharmaceutically acceptable excipient materials in addition to a sweetener composition comprising steviol glycosides, including one or more of compounds SG201-204. Examples of other suitable excipient materials for embodiments of this disclosure include, but are not limited to, other sweetening compounds, antiadherents, binders (e.g., microcrystalline cellulose, gum tragacanth, or gelatin), liquid carriers, coatings, disintegrants, fillers, diluents, softeners, emulsifiers, flavoring agents, coloring agents, adjuvants, lubricants, functional agents (e.g., nutrients), viscosity modifiers, bulking agents, glidiants (e.g., colloidal silicon dioxide) surface active agents, osmotic agents, diluents, or any other non-active ingredient, or combinations thereof. For example, the pharmaceutical compositions of the present disclosure may include excipient materials selected from the group consisting of calcium carbonate, coloring agents, whiteners, preservatives, and flavors, triacetin, magnesium stearate, stearates, natural or artificial flavors, essential oils, plant extracts, fruit essences, gelatins, or combinations thereof.

In one embodiment, an edible gel or edible gel mix comprises a sweetener composition comprising steviol glycosides, including one or more of compounds SG201-204. The edible gel or edible gel mixes can optionally include additives, functional ingredients or combinations thereof. One of compounds SG201-204, or a mixture of two or more of compounds SG201-204 with one or more other steviol glycosides, such as Reb D or Reb M, can constitute a sweetener composition of the present disclosure. However, in many embodiments, a sweetener composition comprises one of compounds SG201, SG202, SG203 or SG204, or a mixture of two or more of compounds SG201-204, with one or more other steviol glycosides, such as Reb D or Reb M and one or more other ingredient(s) that is not a steviol glycoside.

Edible gels are gels that can be eaten by a human or animal. Gels often appear to be solid, jelly-like materials. Non-limiting examples of edible gel compositions for use in particular embodiments include gel desserts, puddings, jellies, pastes, trifles, aspics, marshmallows, gummy candies, or the like. Edible gel mixes generally are powdered or granular solids to which a fluid may be added to form an edible gel composition. Because edible gel products found in the marketplace typically are sweetened with sucrose, it is desirable to sweeten edible gels with an alternative sweetener in order provide a low- calorie or non-calorie alternative.

Non-limiting examples of gelling ingredients for use in particular embodiments include gelatin, alginate, carageenan, gum, pectin, konjac, agar, food acid, rennet, starch, starch derivatives, and combinations thereof. It is well known to those having ordinary skill in the art that the amount of gelling ingredient used in an edible gel mix or an edible gel composition varies considerably depending on a number of factors, such as the particular gelling ingredient used, the particular fluid base used, and the desired properties of the gel.

Edible gel mixes and edible gels may be prepared using other ingredients in addition to the sweetener composition comprising steviol glycosides, including compounds SG201-204, and the gelling agent. Non-limiting examples of other ingredients for use in particular embodiments include a food acid, a salt of a food acid, a buffering system, a bulking agent, a sequestrant, a cross-linking agent, one or more flavors, one or more colors, and combinations thereof.

In one embodiment, a dental composition comprises a sweetener composition comprising steviol glycosides, including one or more of compounds SG201-204. Dental compositions generally comprise an active dental substance and a base material. A sweetener composition comprising steviol glycosides, including one or more of compounds SG201-204, can be used as the base material to sweeten the dental composition. The dental composition may be in the form of any oral composition used in the oral cavity such as mouth freshening agents, gargling agents, mouth rinsing agents, toothpaste, tooth polish, dentifrices, mouth sprays, teeth-whitening agent, dental floss, compositions to treat one or more oral indications (e.g., gingivitis), and the like, for example.

As referred to herein, "active dental substance" means any composition which can be used to improve the aesthetic appearance and/or health of teeth or gums or prevent dental cavities. As referred to herein, "base material" refers to any inactive substance used as a vehicle for an active dental substance, such as any material to facilitate handling, stability, dispersibility, wettability, foaming, and/or release kinetics of an active dental substance.

Suitable active dental substances for embodiments of this disclosure include, but are not limited to, substances which remove dental plaque, remove food from teeth, aid in the elimination and/or masking of halitosis, prevent tooth decay, and prevent gum disease (i.e., Gingiva). Examples of suitable active dental substances for embodiments of the present disclosure include, but are not limited to, anticaries drugs, fluoride, sodium fluoride, sodium monofluorophosphate, stannos fluoride, hydrogen peroxide, carbamide peroxide (i.e., urea peroxide), antibacterial agents, plaque removing agents, stain removers, anticalculus agents, abrasives, baking soda, percarbonates, perborates of alkali and alkaline earth metals, or similar type substances, or combinations thereof. Such components generally are recognized as safe (GRAS) and/or are U.S. Food and Drug Administration (FDA)-approved.

In a particular embodiment, a dental composition comprises a sweetener composition comprising steviol glycosides, including one or more of compounds SG201-204, and an active dental substance. Generally, the amount of the sweetener varies widely depending on the nature of the particular dental composition and the desired degree of sweetness. Those skilled in the art will be able to discern a suitable amount of sweetener for such dental composition. In a particular embodiment, steviol glycosides, including one or more of compounds SG201-204, is present in the dental composition in a total amount in the range of about 1 to about 5,000 ppm of the dental composition and the at least one additive is present in the dental composition in an amount in the range of about 0.1 to about 100,000 ppm of the dental composition.

Foodstuffs include, but are not limited to, confections, condiments, chewing gum, cereal, baked goods, and dairy products.

In one embodiment, a confection comprises a sweetener composition comprising steviol glycosides, including one or more of compounds SG201-204. As referred to herein, "confection" can mean a sweet, a lollie, a confectionery, or similar term. The confection generally contains a base composition component and a sweetener component. A sweetener composition comprising steviol glycosides, including compounds SG201-204 N can serve as the sweetener component. The confection may be in the form of any food that is typically perceived to be rich in sugar or is typically sweet. According to particular embodiments of the present disclosure, the confections may be bakery products such as pastries; desserts such as yogurt, jellies, drinkable jellies, puddings, Bavarian cream, blancmange, cakes, brownies, mousse and the like, sweetened food products eaten at tea time or following meals; frozen foods; cold confections, e. g. types of ice cream such as ice cream, ice milk, lacto-ice and the like (food products in which sweeteners and various other types of raw materials are added to milk products, and the resulting mixture is agitated and frozen), and ice confections such as sherbets, dessert ices and the like (food products in which various other types of raw materials are added to a sugary liquid, and the resulting mixture is agitated and frozen); general confections, e. g., baked confections or steamed confections such as crackers, biscuits, buns with bean- jam filling, halvah, alfajor, and the like; rice cakes and snacks; table top products; general sugar confections such as chewing gum (e.g. including compositions which comprise a substantially water-insoluble, chewable gum base, such as chicle or substitutes thereof, including jetulong, guttakay rubber or certain comestible plant derived or synthetic resins or waxes), hard candy, soft candy, mints, nougat candy, jelly beans, fudge, toffee, taffy, Swiss milk tablet, licorice candy, chocolates, gelatin candies, marshmallow, marzipan, divinity, cotton candy, and the like; sauces including fruit flavored sauces, chocolate sauces and the like; edible gels; crèmes including butter crèmes, flour pastes, whipped cream and the like; jams including strawberry jam, marmalade and the like; and breads including sweet breads and the like or other starch products, and combinations thereof. As referred to herein, "base composition" means any composition which can be a food item and provides a matrix for carrying the sweetener component.

In a particular embodiment, steviol glycosides including one or more of compounds SG201-204, are present in the confection in an amount in the range of about 30 ppm to about 6000 ppm of the confection, or about 1 ppm to about 10,000 ppm of the confection or about 10 ppm to about 5000 ppm, about 500 ppm to about 5000 ppm, about 100 ppm to about 5000 ppm, about 100 ppm to about 7000 ppm, about 200 ppm to about 4000 ppm, about 500 ppm to 7500 ppm, about 1000 ppm to about 8000 ppm, about 2000 ppm to about 5000 ppm, about 3000 ppm to about 7000 ppm or about 4000 ppm to about 6000 ppm of the confection..

In another embodiment, a condiment comprises steviol glycosides, including one or more of compounds SG201-204. In another embodiment a condiment comprises a sweetener composition comprising steviol glycosides, including compounds SG201-204. Condiments, as used herein, are compositions used to enhance or improve the flavor of a food or beverage. Non-limiting examples of condiments include ketchup (catsup); mustard; barbecue sauce; butter; chili sauce; chutney; cocktail sauce; curry; dips; fish sauce; horseradish; hot sauce; jellies, jams, marmalades, or preserves; mayonnaise; peanut butter; relish; remoulade; salad dressings (e.g., oil and vinegar, Caesar, French, ranch, bleu cheese, Russian, Thousand Island, Italian, and balsamic vinaigrette), salsa; sauerkraut; soy sauce; steak sauce; syrups; tartar sauce; and Worcestershire sauce.

In one embodiment, a chewing gum composition comprises a sweetener composition comprising steviol glycosides, including one or more of compounds SG201-204. Chewing gum compositions generally comprise a water-soluble portion and a water-insoluble chewable gum base portion. The water soluble portion, which typically includes the sweetener or sweetener composition, dissipates with a portion of the flavoring agent over a period of time during chewing while the insoluble gum base portion is retained in the mouth. The insoluble gum base generally determines whether a gum is considered chewing gum, bubble gum, or a functional gum.

In a particular embodiment, a chewing gum composition comprises or a sweetener composition comprising steviol glycosides, including one or more of compounds SG201-204 and a gum base. In a particular embodiment, steviol glycosides, including one or more of compounds SG201-204 are present in the chewing gum composition in a total amount in the range of about 1 ppm to about 10,000 ppm of the chewing gum composition.

In one embodiment, a cereal composition comprises a sweetener composition comprising steviol glycosides, including one or more of compounds SG201-204. Cereal compositions typically are eaten either as staple foods or as snacks. Non-limiting examples of cereal compositions for use in particular embodiments include ready-to-eat cereals as well as hot cereals. Ready-to-eat cereals are cereals which may be eaten without further processing (i.e. cooking) by the consumer. Examples of ready-to-eat cereals include breakfast cereals and snack bars. Breakfast cereals typically are processed to produce a shredded, flaky, puffy, or extruded form. Breakfast cereals generally are eaten cold and are often mixed with milk and/or fruit. Snack bars include, for example, energy bars, rice cakes, granola bars, and nutritional bars. Hot cereals generally are cooked, usually in either milk or water, before being eaten. Non-limiting examples of hot cereals include grits, porridge, polenta, rice, and rolled oats.

A sweetener composition comprising steviol glycosides, including one or more of compounds SG201-204, can be added to the cereal composition as a coating, such as, for example, by combining a sweetener comprising the steviol glycosides with a food grade oil and applying the mixture onto the cereal. In a different embodiment, a sweetener composition comprising the steviol glycosides and the food grade oil may be applied to the cereal separately, by applying either the oil or the sweetener first. A sweetener composition comprising steviol glycosides can also be added to the cereal composition as a glaze. Steviol glycosides can be added as a glaze by combining with a glazing agent and a food grade oil or fat and applying the mixture to the cereal. In yet another embodiment, a gum system, such as, for example, gum acacia, carboxymethyl cellulose, or algin, may be added to the glaze to provide structural support. In addition, the glaze also may include a coloring agent, and also may include a flavor. A sweetener composition comprising steviol glycosides can also be added to the cereal composition as a frosting. In one such embodiment, a sweetener composition comprising steviol glycosides is combined with water and a frosting agent and then applied to the cereal.

In a particular embodiment, steviol glycosides are present in the cereal composition in an amount in the range of about 0.02 to about 1.5 weight percent of the cereal composition.

In another embodiment, a baked good comprises a sweetener composition comprising steviol glycosides, including one or more of compounds SG201-204. Baked goods, as used herein, include ready to eat and all ready to bake products, flours, and mixes requiring preparation before serving. Non-limiting examples of baked goods include cakes, crackers, cookies, brownies, muffins, rolls, bagels, donuts, strudels, pastries, croissants, biscuits, bread, bread products, and buns.

Exemplary baked goods can be classified into three groups: bread-type doughs (e.g., white breads, variety breads, soft buns, hard rolls, bagels, pizza dough, and flour tortillas), sweet doughs (e.g., Danishes, croissants, crackers, puff pastry, pie crust, biscuits, and cookies), and batters (e.g., cakes such as sponge, pound, devil's food, cheesecake, and layer cake, donuts or other yeast raised cakes, brownies, and muffins). Doughs generally are characterized as being flour-based, whereas batters are more water-based.

Baked goods in accordance with particular embodiments of this disclosure generally comprise a combination of sweetener, water, and fat. Baked goods made in accordance with many embodiments of this disclosure also contain flour in order to make a dough or a batter. The term "dough" as used herein is a mixture of flour and other ingredients stiff enough to knead or roll. The term "batter" as used herein consists of flour, liquids such as milk or water, and other ingredients, and is thin enough to pour or drop from a spoon.

In one embodiment, a dairy product comprises a sweetener composition comprising steviol glycosides, including one or more of compounds SG201-204. Dairy products and processes for making dairy products suitable for use in this disclosure are well known to those of ordinary skill in the art. Dairy products, as used herein, comprise milk or foodstuffs produced from milk. Non-limiting examples of dairy products suitable for use in embodiments of this disclosure include milk, milk cream, sour cream, crème fraiche, buttermilk, cultured buttermilk, milk powder, condensed milk, evaporated milk, butter, cheese, cottage cheese, cream cheese, yogurt, ice cream, frozen custard, frozen yogurt, gelato, via, piima, filmjölk, kajmak, kephir, viili, kumiss, airag, ice milk, casein, ayran, lassi, khoa, or combinations thereof. Milk is a fluid secreted by the mammary glands of female mammals for the nourishment of their young. The female ability to produce milk is one of the defining characteristics of mammals and provides the primary source of nutrition for newborns before they are able to digest more diverse foods. In particular embodiments of this disclosure, the dairy products are derived from the raw milk of cows, goats, sheep, horses, donkeys, camels, water buffalo, yaks, reindeer, moose, or humans.

In a particularly desirable embodiment, the dairy composition comprises a sweetener composition comprising steviol glycosides, including one or more of compounds SG201-204, in combination with a dairy product. In a particular embodiment, steviol glycosides, including one or more of compounds SG201-204, are present in the dairy composition in a total amount in the range of about 200 to about 20,000 weight percent of the dairy composition.

Tabletop sweetener compositions containing steviol glycosides, including one or more of compounds SG201-204, are also contemplated herein. The tabletop composition can further include a variety of other ingredients, including but not limited to at least one bulking agent, additive, anti-caking agent, functional ingredient or combination thereof.

Suitable "bulking agents" include, but are not limited to, maltodextrin (10 DE, 18 DE, or 5 DE), corn syrup solids (20 or 36 DE), sucrose, fructose, glucose, invert sugar, sorbitol, xylose, ribulose, mannose, xylitol, mannitol, galactitol, erythritol, maltitol, lactitol, isomalt, maltose, tagatose, lactose, inulin, glycerol, propylene glycol, polyols, polydextrose, fructooligosaccharides, cellulose and cellulose derivatives, and the like, and mixtures thereof. Additionally, in accordance with still other embodiments of the disclosure, granulated sugar (sucrose) or other caloric sweeteners such as crystalline fructose, other carbohydrates, or sugar alcohol can be used as a bulking agent due to their provision of good content uniformity without the addition of significant calories.

The tabletop sweetener compositions can be packaged in any form known in the art. Non-limiting forms include, but are not limited to, powder form, granular form, packets, tablets, sachets, pellets, cubes, solids, and liquids. The amount of steviol glycosides, including one or more of compounds SG201-204, in a dry-blend tabletop sweetener formulation can vary. In a particular embodiment, a dry-blend tabletop sweetener formulation may contain steviol glycosides in an amount from about 1 % (w/w) to about 10 % (w/w) of the tabletop sweetener composition.

A tabletop sweetener composition also may be embodied in the form of a liquid, wherein a sweetener composition comprising steviol glycosides, including compounds SG201-204, is combined with a liquid carrier. Suitable non-limiting examples of carrier agents for liquid tabletop functional sweeteners include water, alcohol, polyol, glycerin base or citric acid base dissolved in water, and mixtures thereof.

In one embodiment, the sweetened composition is a beverage product comprising steviol glycosides, including one or more of compounds SG201-204. As used herein a "beverage product" is a ready-to-drink beverage, a beverage concentrate, a beverage syrup, frozen beverage, or a powdered beverage. Suitable ready-to-drink beverages include carbonated and non-carbonated beverages. Carbonated beverages include, but are not limited to, enhanced sparkling beverages, cola, lemon-lime flavored sparkling beverage, orange flavored sparkling beverage, grape flavored sparkling beverage, strawberry flavored sparkling beverage, pineapple flavored sparkling beverage, ginger- ale, soft drinks and root beer. Non-carbonated beverages include, but are not limited to fruit juice, fruit-flavored juice, juice drinks, nectars, vegetable juice, vegetable-flavored juice, sports drinks, energy drinks, enhanced water drinks, enhanced water with vitamins, near water drinks (e.g., water with natural or synthetic flavorants), coconut water, tea type drinks (e.g. black tea, green tea, red tea, oolong tea), coffee, cocoa drink, beverage containing milk components (e.g. milk beverages, coffee containing milk components, cafe au lait, milk tea, fruit milk beverages), beverages containing cereal extracts, smoothies and combinations thereof.

In another embodiment, a beverage contains a sweetener composition containing steviol glycosides, including one or more of compounds SG201-204, wherein the steviol glycosides are present in the beverage in an amount ranging from about 1 ppm to about 10,000 ppm, such as, for example, from about 25 ppm to about 800 ppm. In another embodiment, steviol glycosides are present in the beverage in an amount ranging from about 100 ppm to about 600 ppm. In yet other embodiments, steviol glycosides are present in the beverage in an amount ranging from about 100 to about 200 ppm, from about 100 ppm to about 300 ppm, from about 100 ppm to about 400 ppm, or from about 100 ppm to about 500 ppm. In still another embodiment, steviol glycosides are present in the beverage in an amount ranging from about 300 to about 700 ppm, such as, for example, from about 400 ppm to about 600 ppm. In a particular embodiment, steviol glycosides are present in the beverage in an amount of about 500 ppm.

In one embodiment, the composition is a beverage and the total glycoside content, or the total at Reb A, Reb B, Reb M and/or Reb D, and one or more of SG201-204 in the beverage is about 50 to 1500 ppm, or 100 to 1200 ppm, 200 to 1000 ppm, 300 to 900 ppm, 350 to 800 ppm, 400 to 600 ppm, or 450 to 550 ppm. In one embodiment, steviol glycosides other than Reb D, Reb M, Reb G, Reb O, Reb N, and/or Reb E, other than Reb A, Reb B, Reb D and/or Reb M, or other than Reb M and/or Reb D, such as one or more of compounds SG201-204, are present in a beverage at about at least 0.001 ppm to about 1000 ppm, e.g., about 1 to 800 ppm, 1 to 600 ppm, 1 to 500 ppm, 50 ppm to 500 ppm, 10 to 100 ppm, 100 to 600 ppm, 200 to 500 ppm, 300 to 400 ppm, 0.1 to 10 ppm, or 0.1 to 50 ppm, including at least 0.001, 0.01, 0.1, 1, 5, 10, 20, 30, 40, 50, 125, 150, 150, 175, or 200 ppm. In one embodiment, steviol glycosides other than Reb D, Reb M, Reb G, Reb O, Reb N, and/or Reb E, other than Reb A, Reb B, Reb D and/or Reb M, or other than Reb M and/or Reb B, compounds such as one or more of compounds SG201-204, are present in a beverage at about 1 to 600 ppm, 10 to 400, 50 to 200, 75 to 150, 5 to 200, 10 to 100, 20 to 90, 30 to 80 ppm, and the like.

Examples of frozen beverages, include, but are not limited to, icees, frozen cocktails, daiquiris, pina coladas, margaritas, milk shakes, frozen coffees, frozen lemonades, granitas, and slushees.

Beverage concentrates and beverage syrups can be prepared with an initial volume of liquid matrix (e.g., water) and the desired beverage ingredients. Full strength beverages are then prepared by adding further volumes of water. Powdered beverages are prepared by dry-mixing all of the beverage ingredients in the absence of a liquid matrix. Full strength beverages are then prepared by adding the full volume of water.

In one embodiment, a beverage contains a sweetener composition comprising steviol glycosides, including one or more of compounds SG201-204. Any sweetener composition comprising steviol glycosides, including one or more of compounds SG201-204 detailed herein can be used in the beverages. In another embodiment, a method of preparing a beverage comprises combining a liquid matrix and steviol glycosides, including one or more of compounds SG201-204. The method can further comprise addition of one or more sweeteners, additives and/or functional ingredients. In still another embodiment, a method of preparing a beverage comprises combining a liquid matrix and a sweetener composition comprising steviol glycosides, including one or more of compounds SG201-204.

A method for imparting a more sugar-like temporal profile, flavor profile, or both to a sweetenable composition comprises combining a sweetenable composition with the sweetener compositions of the present disclosure, i.e., sweetener compositions containing steviol glycosides, including one or more of compounds SG201-204.

The method can further include the addition of other sweeteners, additives, functional ingredients and combinations thereof. Any sweetener, additive or functional ingredient detailed herein can be used.

As used herein, the "sugar-like" characteristics include any characteristic similar to that of sucrose and include, but are not limited to, maximal response, flavor profile, temporal profile, adaptation behavior, mouthfeel, concentration/response function, tastant/and flavor/sweet taste interactions, spatial pattern selectivity, and temperature effects.

In certain embodiments, an agglomerate of steviol glycosides, including compounds SG201-204, sweetener composition is provided. As used herein, "sweetener agglomerate" means a plurality of sweetener particles clustered and held together. Examples of sweetener agglomerates include, but are not limited to, binder held agglomerates, extrudates, and granules. Methods for making agglomerates are known to those of ordinary skill in the art, and are disclosed in more detail in U.S. Patent 6,180,157. Generally described, the process for preparing an agglomerate in accordance with a certain embodiment comprises the steps of preparing a premix solution comprising steviol glycosides, including one or more of compounds SG201-204, sweetener composition and a binding agent in a solvent, heating the premix to a temperature sufficient to effectively form a mixture of the premix, applying the premix onto a fluidized carrier by a fluid bed agglomerator, and drying the resulting agglomerate. The sweetness level of the resulting agglomerate may be modified by varying the amount of the sweetener composition in the premix solution.

In some embodiments provided are substantially dustless and substantially free-flowing extrudates or extruded agglomerates of steviol glycosides, including compounds SG201-204, for a sweetener composition. Such particles may be formed with or without the use of binders using extrusion and spheronization processes.

"Extrudates" or "extruded sweetener composition", as used herein, refers to cylindrical, free- flowing, relatively non-dusty, mechanically strong granules of steviol glycosides, including one or more of compounds SG201-204. The terms "spheres" or "spheronized sweetener composition", as used herein, refer to relatively spherical, smooth, free-flowing, relatively non-dusty, mechanically strong granules. A process for making extrudates is described in U.S. Patent 6,365,216.

In another embodiment, granulated forms of steviol glycosides, including one or more of compounds SG201-204 are provided. As used herein, the terms "granules," "granulated forms," and "granular forms" are synonymous and refer to free-flowing, substantially non-dusty, mechanically strong agglomerates of the steviol glycoside sweetener composition. Methods of granulation are known to those of ordinary skill in the art and are described in more detail in the PCT Publication WO2001/060842.

### Example 1

### Fermentation for Steviol Glycoside Production Including Compounds SG201-204

Steviol glycoside compounds, including compounds SG201-204, Reb D and Reb M, were produced by genetically engineered *Saccharomyces cerevisiae. Saccharomyces* strains EFSC 3261 and EFSC 3841 are described in International Application No. WO 2014/122227.

Fed-batch fermentation was carried out aerobically in 2L (working volume) fermenters which included an about 16 hour growth phase in the base medium (minimal medium containing glucose, ammonium sulfate, trace metals, vitamins, salts, and buffer) followed by about 100 hours of feeding with a glucose-containing defined feed medium. Glucose was utilized as the carbon and energy source and combined with trace metals, vitamins, and salts. The pH was kept near pH 5 and the temperature set-point was 30°C. The feed rate was controlled to prevent oxygen depletion and to minimize ethanol formation (glucose-limited conditions). The fermentation minimal medium is based on Verduyn C, Postma E, Scheffers WA, Van Dijken JP.(1992).Yeast 8, 501-517.

### Isomer Isolation Procedure from Fermentation Broth

Methanol was added to fermentation broth to make a solution that was 55 % methanol (v/v). The resulting solution was thoroughly mixed for approximately 30 minutes. The solution was filtered utilizing a Buchner funnel through a 0.45 µm nylon filter paper to clarify. The filtrate was then dried under nitrogen at RT to complete dryness. The resulting precipitate was solubilized in 20% ethanol (v/v) and purified on an Agilent 1260 semi-preparative HPLC instrument utilizing a Phenomenex Kinetex^{®} XB-C18 5 µm column. An ultrapure water (18.2 MΩ) and methanol gradient was used to separate the desired isomer from the matrix and desired purity was achieved through an iterative approach of compound purification. All similar fractions were pooled and dried under nitrogen at RT prior to any further processing.

### Example 2

### NMR Spectroscopy

All NMR spectra were acquired on a 800MHz Bruker Avance machine (800 MHz for 1H, 201 MHz for 13C) equipped with a cryogenic probe (5 mm CPTCI 1H-13C/15N/D Z-GRD Z44909/0010). Samples were dissolved in conventional solvents, DMSO-d6/D2O 1:1, and D2O, measurement were made at 25 °C. or at 40 °C.

Structures were solved by means of standard homo- and heteronuclear multipulse NMR experiments, namely ¹H,¹H-COSY. ¹H,¹H-TCOSY, ¹H,¹H-ROESY, ¹H,¹³C-HSQC and ¹H,¹³C-HMBC.

### Example 3

### Sensory Testing

Steviol glycoside samples (SG201, SG203, and SG204) were isolated and purified from fermentation broth, purified Reb A and B are of leaf origin, and Reb M is chemically synthesized. All samples were dissolved in reverse osmosis water at the concentrations indicated. Approximately 2-3 milliliters of each solution was tasted and sensory evaluations conducted by two to three experienced tasters. Sensory results are listed in Table 1. SG201, SG203, and SG204 in water do not exhibit sweetness similar to Reb M in water, however SG201, SG203, and SG204 can be used to modify sensory characteristics, including but not limited to sweetness and bitterness, of a sweetener composition or of other materials.

**Table 1**

| Sample | mass (mg) | mass (g) | Actual H2O mass (g) | Actual ppm sample in solution | Sensory Comments |
|---|---|---|---|---|---|
| Reb A | 3.8 | 0.0038 | 12.6937 | 299.4 | some sweet, bitter |
| Reb D | 2.7 | 0.0027 | 8.9670 | 301.1 | better sweetness, less bitter |
| Reb M FCC | 2.9 | 0.0029 | 9.6846 | 299.4 | good sweetness, almost sucrose-like sensation in mouth |
| SG204 | 3.3 | 0.0033 | 11.0002 | 300.0 | bitter, not very sweet |
| SG203 | 3.1 | 0.0031 | 10.3316 | 300.1 | not sweet, meaty, brothy, strecker, methional |
| SG201 | dust | 0.003 | 2.0052 | 300 | not sweet, meaty, brothy, strecker, methional |

## Claims

1. A method of making a sweetener composition suitable for oral ingestion or oral use, comprising: adding a sensory modifying amount of one or more of compounds SG201, SG203, or SG204 according to the following structures: 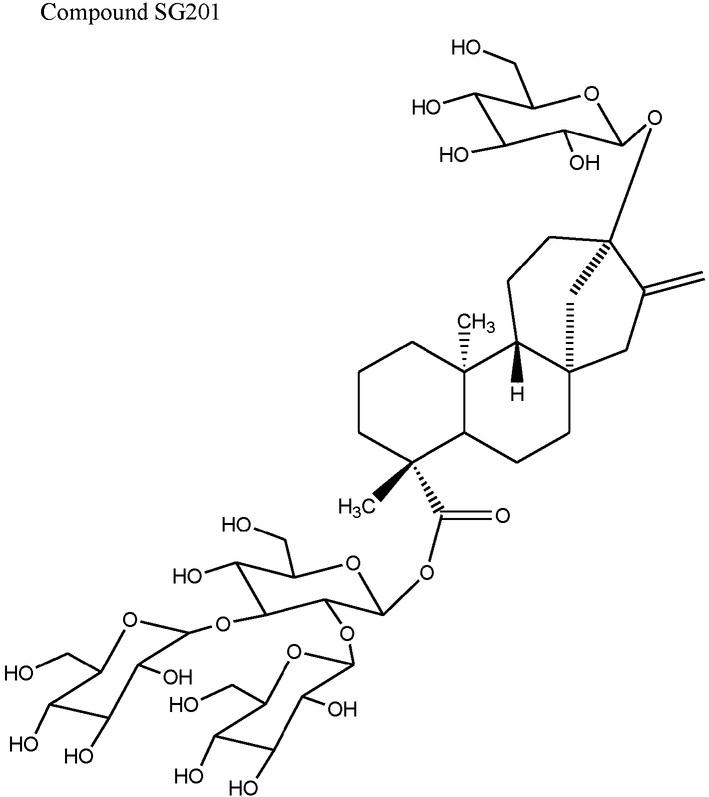 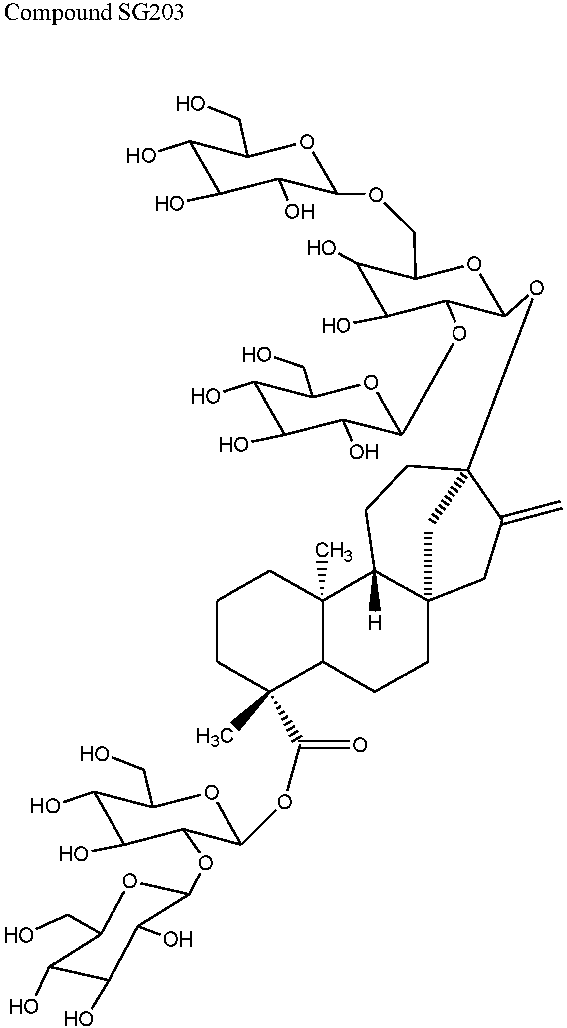 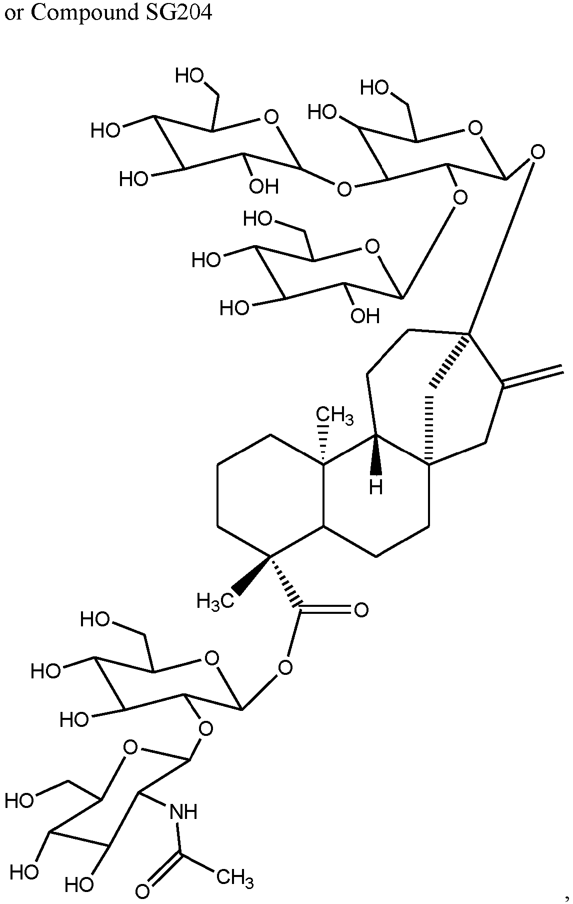 and an amount of one or more of Reb M, Reb D, Reb B or Reb A, wherein a sensory modifying amount of one or more of compounds SG201, SG203, or SG204 is in the range of 0.05 % to 10 % (wt) of a total amount of steviol glycosides in the composition.

2. The method of claim 1, comprising adding a sensory modifying amount of two or more of compounds SG201, SG203, or SG204.

3. The method of any one of claims 1-2, comprising adding an amount of two or more of Reb M, Reb D, Reb B or Reb A.

4. The method of any one of claims 1-3, wherein the composition has Reb M, Reb D or a combination of Reb M and Reb D.

5. The method of any one of claims 1-4, wherein the composition has Reb A.

6. The method of any one of claims 1-5, wherein the sensory modifying amount of one or more of SG201, SG203, or SG204 enhances at least one of roundedness, temporal onset, tailing, sweetness, stringency, or a flavor note.

7. The method of any one of claims 1-6, wherein at least one of compounds SG201, SG203, or SG204 is in an amount that has a sucrose equivalent value of less than about 1.5.

8. The method of any one of claims 1-7, wherein Reb M or Reb D, or a combination thereof, is present in an amount greater than any one of compounds SG201, SG203, or SG204.

9. The method of any one of claims 1-8, wherein the composition is a sweetener composition, a beverage, or a throw syrup.

## Patentansprüche

1. Verfahren zum Herstellen einer Süßstoffzusammensetzung, die für die orale Einnahme oder orale Anwendung geeignet ist, umfassend: Zugabe einer sensorisch modifizierenden Menge einer oder mehrerer Verbindungen SG201, SG203 oder SG204 gemäß den folgenden Strukturen: Verbindung SG201 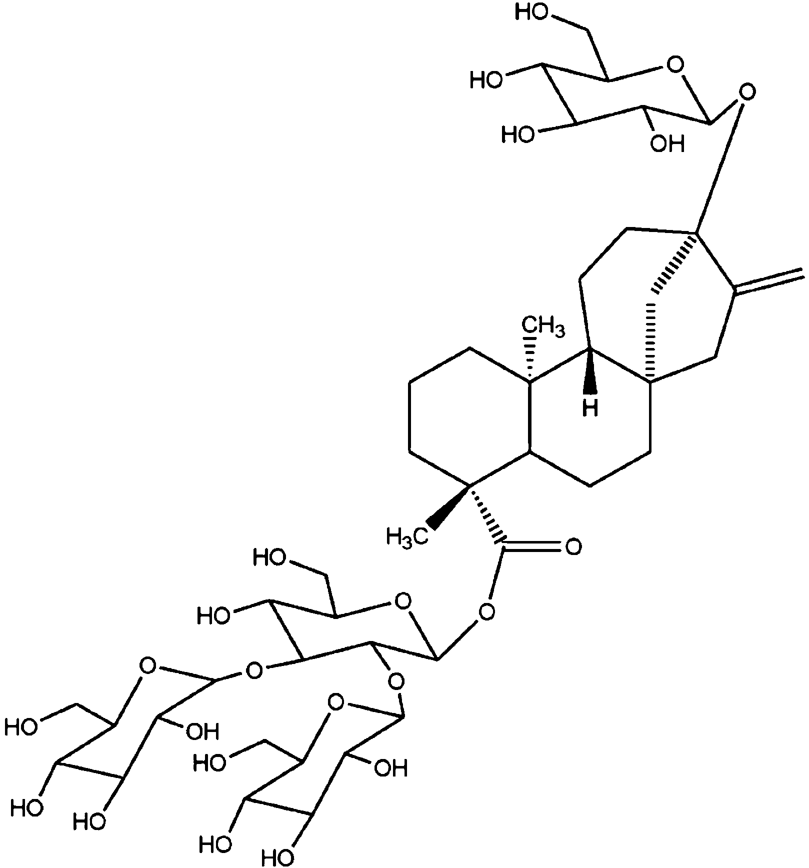 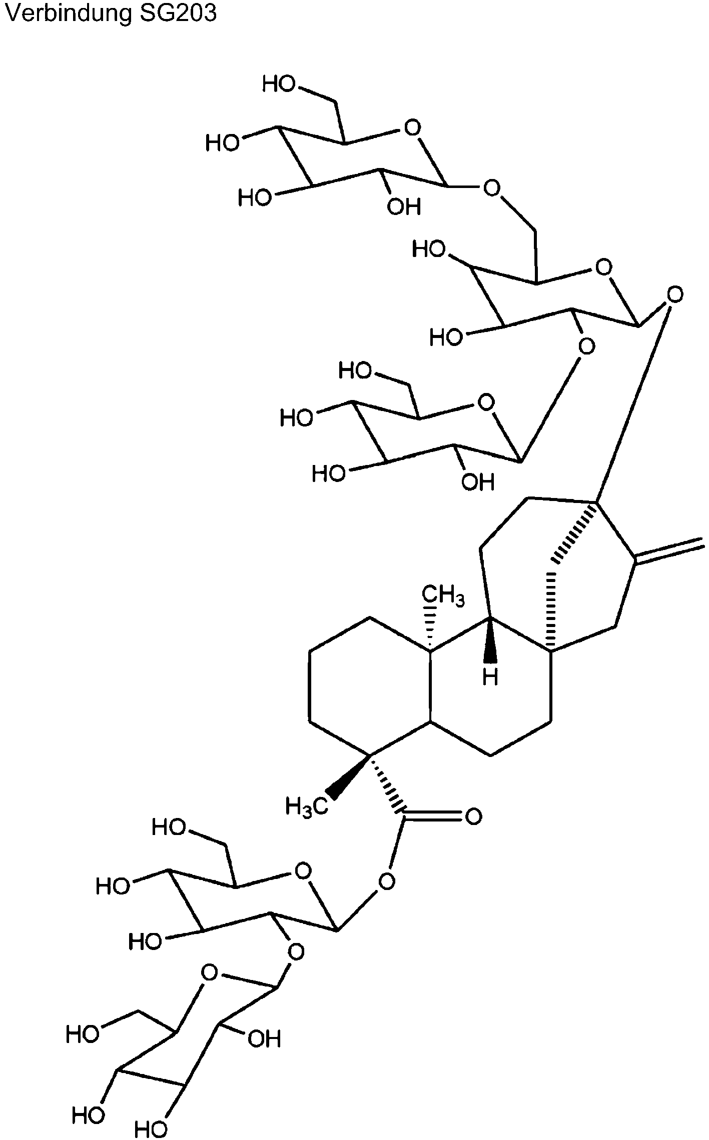 oder und eine Menge von einem oder mehreren von Reb M, Reb D, Reb B oder Reb A, wobei eine sensorisch modifizierende Menge von einer oder mehreren der Verbindungen SG201, SG203 oder SG204 im Bereich von 0,05 Gew.-% bis 10 Gew.-% einer Gesamtmenge an Steviolglycosiden in der Zusammensetzung liegt.

2. Verfahren nach Anspruch 1, umfassend die Zugabe einer sensorisch modifizierenden Menge von zwei oder mehr der Verbindungen SG201, SG203 oder SG204.

3. Verfahren nach einem der Ansprüche 1 bis 2, umfassend die Zugabe einer Menge von zwei oder mehr von Reb M, Reb D, Reb B oder Reb A.

4. Verfahren nach einem der Ansprüche 1 bis 3, wobei die Zusammensetzung Reb M, Reb D oder eine Kombination von Reb M und Reb D aufweist.

5. Verfahren nach einem der Ansprüche 1 bis 4, wobei die Zusammensetzung Reb A aufweist.

6. Verfahren nach einem der Ansprüche 1 bis 5, wobei die sensorisch modifizierende Menge von einer oder mehreren von SG201, SG203 oder SG204 mindestens eines von Rundheit, zeitlichem Einsetzen, Nachklingen, Süße, Stringenz oder eine Geschmacksnote verstärkt.

7. Verfahren nach einem der Ansprüche 1 bis 6, wobei mindestens eine der Verbindungen SG201, SG203 oder SG204 in einer Menge vorliegt, die einen Saccharoseäquivalentwert von weniger als etwa 1,5 aufweist.

8. Verfahren nach einem der Ansprüche 1 bis 7, wobei Reb M oder Reb D oder eine Kombination davon in einer Menge vorhanden ist, die größer ist als die einer der Verbindungen SG201, SG203 oder SG204.

9. Verfahren nach einem der Ansprüche 1 bis 8, wobei die Zusammensetzung eine Süßstoffzusammensetzung, ein Getränk oder ein konzentrierter Sirup ist.

## Revendications

1. Procédé de fabrication d'une composition d'édulcorant appropriée pour une ingestion orale ou un usage oral, comprenant : l'ajout d'une quantité de modification sensorielle d'un ou plusieurs des composés SG201, SG203 ou SG204 selon les structures suivantes : 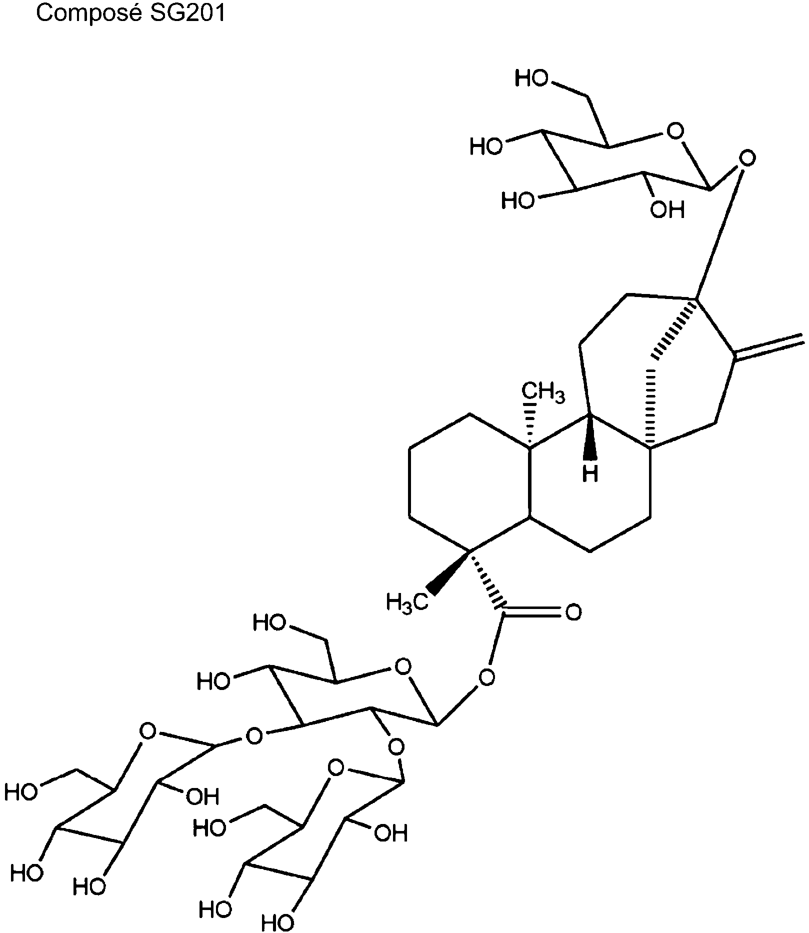 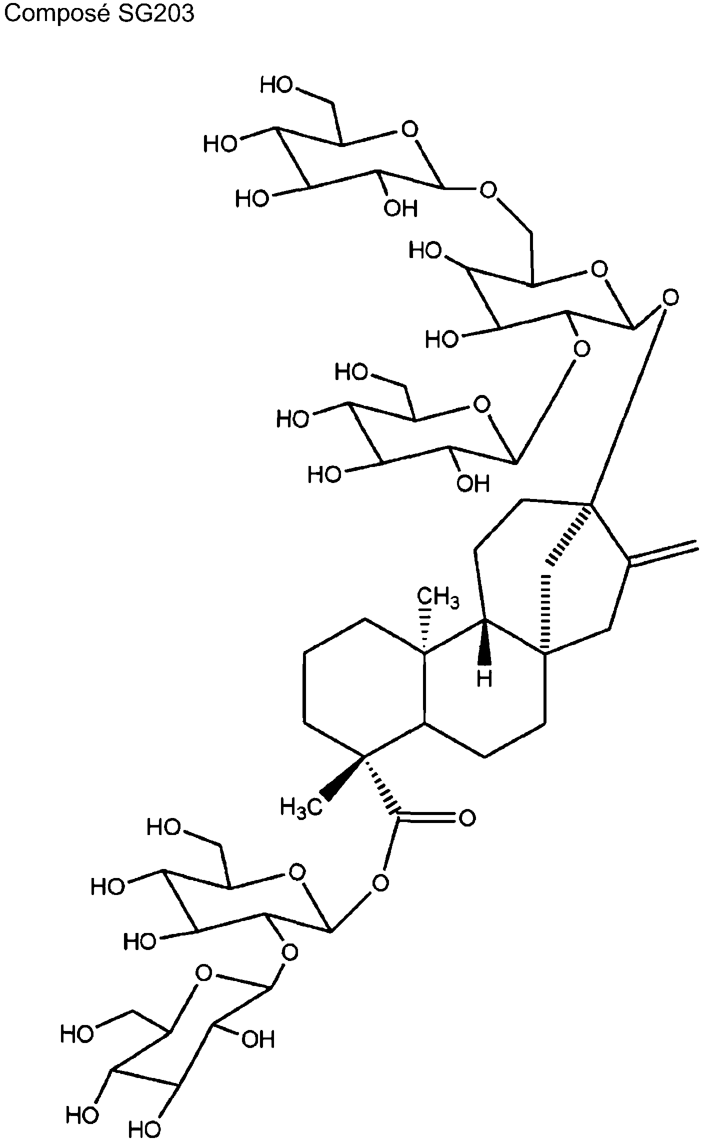 ou 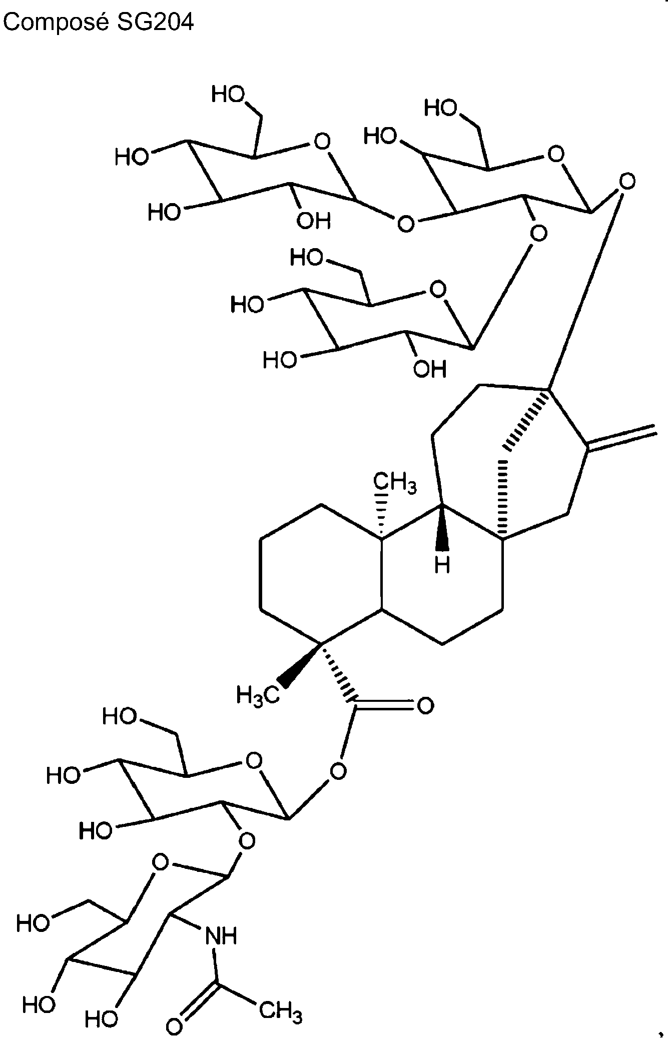 et une quantité d'un ou plusieurs parmi Reb M, Reb D, Reb B ou Reb A, dans lequel une quantité de modification sensorielle d'un ou plusieurs des composés SG201, SG203 ou SG204 est comprise entre 0,05 % et 10 % (en poids) d'une quantité totale de glycosides de stéviol dans la composition.

2. Procédé selon la revendication 1, comprenant l'ajout d'une quantité de modification sensorielle de deux composés ou plus SG201, SG203 ou SG204.

3. Procédé selon l'une quelconque des revendications 1 à 2, comprenant l'ajout d'une quantité de deux ou plus parmi Reb M, Reb D, Reb B ou Reb A.

4. Procédé selon l'une quelconque des revendications 1 à 3, dans lequel la composition a Reb M, Reb D ou une combinaison de Reb M et Reb D.

5. Procédé selon l'une quelconque des revendications 1 à 4, dans lequel la composition a Reb A.

6. Procédé selon l'une quelconque des revendications 1 à 5, dans lequel la quantité de modification sensorielle d'un ou plusieurs parmi SG201, SG203 ou SG204 améliore au moins un parmi la rondeur, le début temporel, la traînée, la douceur, la rigueur ou une note aromatique.

7. Procédé selon l'une quelconque des revendications 1 à 6, dans lequel au moins l'un des composés SG201, SG203 ou SG204 se trouve dans une quantité qui a une valeur d'équivalent saccharose inférieure à environ 1,5.

8. Procédé selon l'une quelconque des revendications 1 à 7, dans lequel Reb M ou Reb D, ou une combinaison de ceux-ci, est présent en une quantité supérieure à l'un quelconque des composés SG201, SG203 ou SG204.

9. Procédé selon l'une quelconque des revendications 1 à 8, dans lequel la composition est une composition d'édulcorant, une boisson ou un sirop jeté.
